(19) Europäisches Patentamt

European Patent Office

Office européen des brevets

(11) **EP 1 494 156 A2**

(12) **EUROPEAN PATENT APPLICATION**

(43) Date of publication:
**05.01.2005 Bulletin 2005/01**

(51) Int Cl.⁷: **G06F 17/60**

(21) Application number: **04103140.2**

(22) Date of filing: **02.07.2004**

(84) Designated Contracting States:
**AT BE BG CH CY CZ DE DK EE ES FI FR GB GR
HU IE IT LI LU MC NL PL PT RO SE SI SK TR**
Designated Extension States:
**AL HR LT LV MK**

(30) Priority: **04.07.2003 FI 20031020
16.02.2004 FI 20045040**

(71) Applicant: **Medicel Oy
00290 Helsinki (FI)**

(72) Inventors:
• **Varpela, Pertteli
02730, Espoo (FI)**
• **Pellikka, Tarmo
01800, Klaukkala (FI)**
• **Kolmer, Meelis
02660, Espoo (FI)**

(74) Representative: **Virkkala, Jukka Antero
Kolster Oy Ab,
Iso Roobertinkatu 23,
P.O. Box 148
00121 Helsinki (FI)**

(54) **Information management system for managing workflows**

(57)     An information management system for managing workflows comprises a syntactic and semantic data entity type definition (1214) for each data entity type, and a data entity definition (1216) for each data entity (1250), each of which contains a subset of the information to be processed by a workflow. Tool definitions (1208) define tools (1254) that execute data processing tasks in a tool server (1222). Tool input binders (1210) and tool output binders (1212) bind tool inputs or outputs to a specific data entity type. A workflow definition (1202, 1202') comprises one or more workflow input definitions (1204, 1204') and workflow output definitions (1206, 1206'). The workflow input and output definitions collectively define a data flow network from the input of the workflow to the output of the workflow via one or more instances of tool definitions (1208).

Fig. 12D

**Description**

BACKGROUND OF THE INVENTION

The invention relates to an information management system ("IMS" in short) for managing workflows. As used herein, a workflow is a well-defined set of data operations (briefly: works). The invention finds particular use in connection with biochemical information.

Biochemical research has experienced tremendous growth recently. As a consequence, many workflows and experiments more or less improvised and the discipline lacks off-the-shelf software tools for managing complex workflows and experiments. A first problem resulting from the lack of suitable software tools is that experiments that require several steps and/or information processing tools may be difficult to reproduce, with or without modifications.

In the prior art, such multi-steps complex workflows have been automated with batch or script files, that typically have the following format:

*<tool_name> <input_file> <output_file> <parameter_1> ... <parameter_n>*.

For example, a line in a script file, such as:

*digest -sequence my_in_file -out_file my_out_file -auto -unfavoured*

...would instruct the tool named digest to process the named input file controlled by parameters *"-auto"* and *"-unfavoured"*, and to save the result in the named output file. Such script files suffer from numerous disadvantages. For instance, script file writing requires programming skills, which are seldom possessed by researchers in the biochemical field or other areas not directly related to programming.

A second problem is that known IMS systems typically expect to receive data in specific formats, which is why they provide poor support in a field such as biochemistry, in which discipline-wide standards for presenting information do not exist. Biochemical research brings tremendous amounts of data at a rate which has never been seen in any discipline of science. A problem underlying the invention relates to the difficulties in organizing vast amounts of rapidly-varying information. IMS systems can be free-form or structured. A well-known example of a free-form IMS is a local-area network of a research institute, in which information producers (researches or the like) can enter information in an arbitrary format, using any of the commonly-available or proprietary applications programs, such as word processors, spreadsheets, databases etc. A structured IMS means a system with system-wide rules for storing information in a unified database.

BRIEF DESCRIPTION OF THE INVENTION

An object of the present invention is to provide an information management system (later abbreviated as "IMS") so as to solve the first problem. In other words, the object of the invention is to provide an IMS for managing workflows and software tools. The object of the invention is achieved by an IMS which is characterized by what is stated in the independent claims. The preferred embodiments of the invention are disclosed in the dependent claims.

An IMS according to the invention is able to manage workflows, wherein each workflow defines an ordered set of one or more data processing tasks that relate to information. The IMS comprises a workflow manager, which comprises:

a data entity type definition for each of several data entity types, wherein each data entity type definition relates to syntax and semantics of data;

a data entity definition for each of several data entities, wherein each data entity relates to a specific data entity type and contains a subset of the information;

- a tool definition for each of several tools, wherein each tool is capable of executing a subset of the data processing tasks;
- one or more tool servers for executing the tools;
- a set of tool input binders and tool output binders, each of which binds an input or output, respectively, of a tool to a specific data entity type;
- a workflow definition for each of several workflows, wherein each workflow definition comprises one or more workflow input definitions, each workflow input definition indicating one or more data entities as an input of the workflow; and one or more workflow output definitions, each workflow output definition indicating one or more data entities as an output of the workflow;
- wherein the workflow input definitions and workflow output definitions collectively define a data flow network from the input of the workflow to the output of the workflow via one or more instances of tool definitions.

The invention is based on the use of tool input and output binders that connect each tool to specific data entity or data entity type, thereby providing a possibility to create work instances with work inputs and outputs that connect work instances to specific data entities that satisfy the required types of data entities.

The data entity types provide syntactic and/or semantic information for type checking of data entities that are to be connected to child workflows which involve a specific set of the tools. The type check is based on tool input binders and tool output binders.

The work inputs and outputs collectively define a data flow network from the workflow's input to its output through works that are done by executing specific tools. The inputs and outputs of the tools are connected to data entities via an appropriate type check, so that data integrity is ensured. The work inputs and outputs that define the workflow are preferably created and updated via a graphical user interface that provides drag-and-drop functionality. Unlike a conventional drag-and-drop interface, which causes an immediate execution of a specified software tool, the IMS according to the invention creates workflow input definitions and workflow output definitions, which collectively define a data flow network from the input of the workflow to the output of the workflow via one or more instances of tool definitions. The workflow may be executed in response to a user input via the graphical user interface When the workflow is executed the data flow network defines the order of execution of the tools, such that a first tool whose output is an input of a second tool is executed prior to the second tool Thus a set or processing tasks of virtually any complexity can be executed automatically without further user interaction. The data flow network that specifies the workflow's input and output, via the tools, is stored in database tables or the like, whereby the workflows are easily repeated, with or without modifications, and traced, should the need to repeat the workflows arise.

In addition to defining the set of tools that are used when the workflow is executed, a workflow definition may also comprise documenting descriptions to assist documentation of the workflow.

A preferred embodiment of the invention relates to an IMS that solves the second problem. In other words, the preferred embodiment should provide an IMS that is logically complete so that as little external information as possible is needed to interpret the information contained in the IMS. In addition, the information contained in the IMS should be structured, so that the information can be accessed by a wide variety of information processing tools.

Such an IMS may be used to store information about populations, individuals, reagents or samples of other biomaterials (anything that can be studied as a biological/biochemical system or its component). The IMS preferably comprises an experiment database. An experiment can be a real-life experiment ("wet lab") or a simulated experiment ("in-silico"). According to the invention, both experiment types produce data sets, such that each data set comprises:

- a variable value matrix for describing variable values in a row-column organization;
- a row description list, in a variable description language, of the rows in the variable value matrix;
- a column description list, in a variable description language, of the columns in the variable value matrix;
- a fixed dimension description, in a variable description language, of one or more fixed dimensions that are common to all values in the variable value matrix.

According to this preferred embodiment of the invention, the numerical values of each experiment are stored, as scalar numbers, in a variable value matrix having a row-column organization. Such row-column matrixes can be further processed with a wide variety of off-the-shelf or proprietary application programs. There are separate row and column description lists for describing, respectively, the meaning of the rows and columns in the variable value matrix. A separate fixed dimension description describes the fixed dimensions that are common to all values in the variable value matrix. The row and column description lists, as well as the fixed dimension description, are written in a variable description language in order to link arbitrary variable values to the structured information of the IMS.

A benefit achieved by the use of the variable description language (=VDL) is that the IMS is largely self-sufficient. Little or no external information is needed to interpret the numerical values. Also, it is a relatively straightforward task to force an automated syntax check on the variable expressions. An essential feature of the VDL is that it permits the description of variables in varying detail level. For example, the VDL may describe a variable in terms of biomaterial (population - individual - sample; organism - organ - tissue, cell type, etc.), physical quantities and time, but we may omit details that are not essential to our current context.

XML (eXtendible Markup Language) is a well-known example of a language that can be used as a variable description language. A problem with XML is, however, that it is intended to describe virtually any structured information, which results in lengthy expressions that are poorly readable by humans. Accordingly, a preferred embodiment of the invention relates to a variable description language that is better suited to describing biological variables than XML is. Also, expressions in XML and its biological or mathematical variants, such as SBML (Systems Biology Markup Language) or CellML (Cell Markup Language) or MathML (Mathematical Markup Language), are generally too long or complex to serve as self-documenting symbols for describing biological variables in mathematical models. Accordingly, a further preferred embodiment of the invention comprises a compact but extendible VDL that overcomes the problems of XML and its variants.

A benefit achieved by storing the numerical values as a scalar matrix is that the matrix can be analyzed with many commercially available data-mining tools, such as self-organizing maps or other clustering algorithms, that do not readily process dimensioned values. Accordingly, the row and column descriptions are stored separately. A benefit achieved by the use of a third list, namely the fixed dimension description, is that dimensions common to rows and columns need not be duplicated in the row and column description lists.

The processing speed of the IMS can be increased by storing each data set (each data set comprising a variable value matrix, row and column description lists and a fixed dimension description) as a container for data, and storing only an address or identifier of the container in a database. Assuming that SQL (structured query language) or other database queries are used to retrieve the data sets, the single-container approach dramatically reduces the number of individual data items to be processed by SQL queries. When individual data elements are needed, the entire container can be processed with a suitable tool, such as a spreadsheet or flat-file database system.

According to another preferred embodiment of the invention, the IMS further comprises a biochemical entity database containing objects or tables. The variable description language comprises variable descriptions, each variable description comprising one or more pairs of keyword and name. For each object or table of the biochemical entity database, there is a keyword that references that object or table. This embodiment facilitates automated syntax or other checks made to information to be stored.

A further advantage of the data sets according to the invention is good support for well-defined contexts. A context defines the scope of an experiment, either wet-lab or in-silico. Each context is defined in terms of biomaterials, variables and time.

BRIEF DESCRIPTION OF THE DRAWINGS

In the following the invention will be described in greater detail by means of preferred embodiments with reference to the attached drawings, in which

Figure 1 is a block diagram of an IMS in which the invention can be used;
Figure 2 is an entity-relationship model of a database structure of the IMS;
Figures 3A and 3B illustrate a preferred variable description la nguage, or VDL;
Figure 3C illustrates a syntax-checking process for a variable expression in the VDL;
Figure 4 shows examples of compound variable expressions in the VDL;
Figure 5 shows how the VDL can be used to express different data contexts;
Figures 6A to 6C illustrate data sets according to various preferred embodiments of the invention;
Figure 7A is a block diagram of a pathway as stored in the IMS;
Figure 7B shows an example of complex pathway that contains simpler pathways;
Figure 7C shows an example of pathway that relates to analogue and Boolean flux rate equations;
Figure 8 shows a visualized form of a pathway;
Figure 9A shows an experiment object in an experiments section of the IMS;
Figure 9B illustrates creation of a project plan from a set of desired results;
Figure 10 shows an example of an object-based implementation of the biomaterials section of the IMS;
Figures 11A and 11 B demonstrate data traceability in the light of two examples;
Figure 12A shows an information-entity relationship for describing and managing complex workflows within the IMS;
Figure 12B shows a client-server architecture comprising a graphical workflow editor being executed in a client terminal;
Figure 12C shows how the workflow editor can represent workflows as a network of tools and data entities, such that data entities are inputs or outputs of tools;
Figure 12D shows an enhanced version of the information-entity relationship shown in Figure 12A;
Figure 13 shows an exemplary user interface for a workflow manager;
Figure 14 shows an object-based implementation of the invention;
Figure 15 shows a procedure for starting a tool server;
Figures 16A and 16B show a tool definition file;
Figure 16C shows a script generated automatically under control of tool definition information;
Figures 17A and 17B show a class diagram for an exemplary tool execution environment; and
Figure 18 shows process steps to execute a workflow.

DETAILED DESCRIPTION OF THE INVENTION

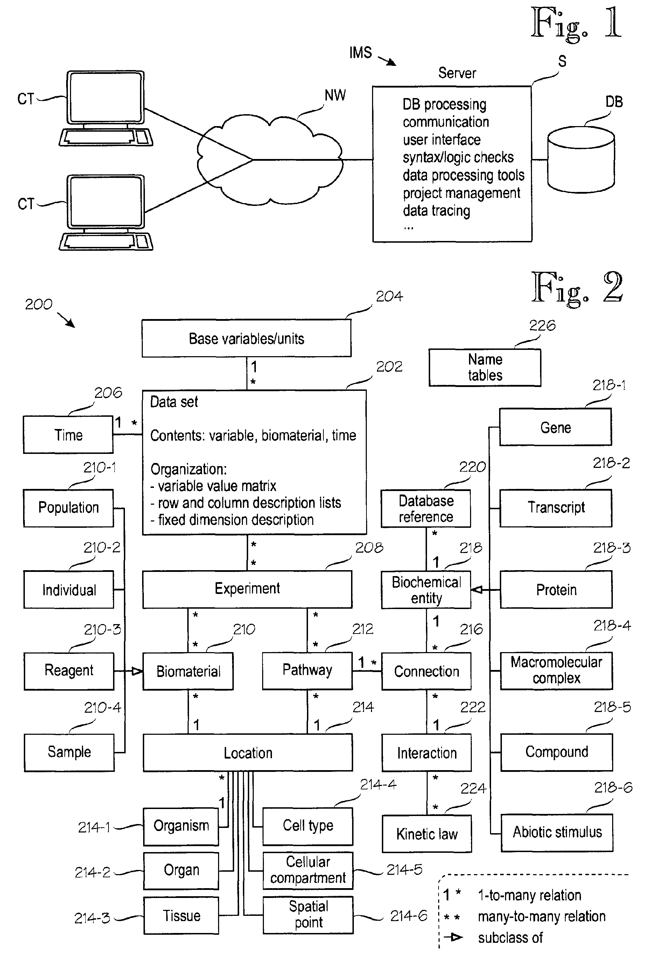

Figure 1 is a simplified block diagram of an information management system IMS in which the invention can be used. In this example, the IMS is implemented as a client/server system. Several client terminals CT, such as graphical workstations, access a server (or set or servers) S via a network NW, such as a local-area network or the Internet. The server comprises or is connected to a database DB. The information processing logic within the server and the data within the database constitute the IMS. The database DB is comprised of structure and content. A preferred embodiment of the invention provides improvements to the structure of the database DB of the IMS. The server S also comprises various processing logics. A communication logic provides the basic server functions for communicating with the client terminals. There is preferably a user interface logic for creating various user interfaces. There may be various checks for checking the meaningfulness (such as syntax or range checks) of data to be entered. A very useful feature is a project manager with a tracing logic that provides visual tracing of data.

The server (or set of servers) S also comprises various data processing tools for data analysis, visualization, data mining, etc. A benefit of storing the data sets as containers in a row-column organization (instead of addressing each data item separately by SQL queries) is that such data sets of rows and columns can easily be processed with commercially available analysis or visualization tools. Before describing embodiments for the actual invention, i.e., the IMS for managing workflows and software tools, preferred embodiments for describing biochemical data will be described in connection with Figures 2 to 11 B. Detailed embodiments of the IMS for managing workflows and software tools will be described in connection with Figures 12A to 18.

**Data sets**

Figure 2 is an entity-relationship model of a database structure 200 of the IMS. The database structure 200 comprises the following major sections: base variables/units 204, data sets 202, experiments 208, biomaterials 210, pathways 212 and, optionally, locations 214.

Data sets 202 describe the numerical values stored in the IMS. Each data set is comprised of a variable set, biomaterial information and time organized in

- a variable value matrix for describing variable values in a row-column organization;
- a row description list, in a variable description language, of the rows in the variable value matrix;
- a column description list, in a variable description language, of the columns in the variable value matrix; and
- a fixed dimension description, in a variable description language, of one or more fixed dimensions that are common to all values in the variable value matrix.

The variable description language binds syntactical elements and semantic objects of the information model together, by describing what is quantified in terms of variables (eg count, mass, concentration), units (eg pieces, kg, mol/l), biochemical entities (eg specific transcript, specific protein, specific compound) and a location where the quantification is valid (eg human_eyelid_epith_nuc) in a multi-level location hierarchy of biomaterials (eg environment, population, individual, reagent, sample, organism, organ, tissue, cell type) and relevant expressions of time when the quantification is valid.

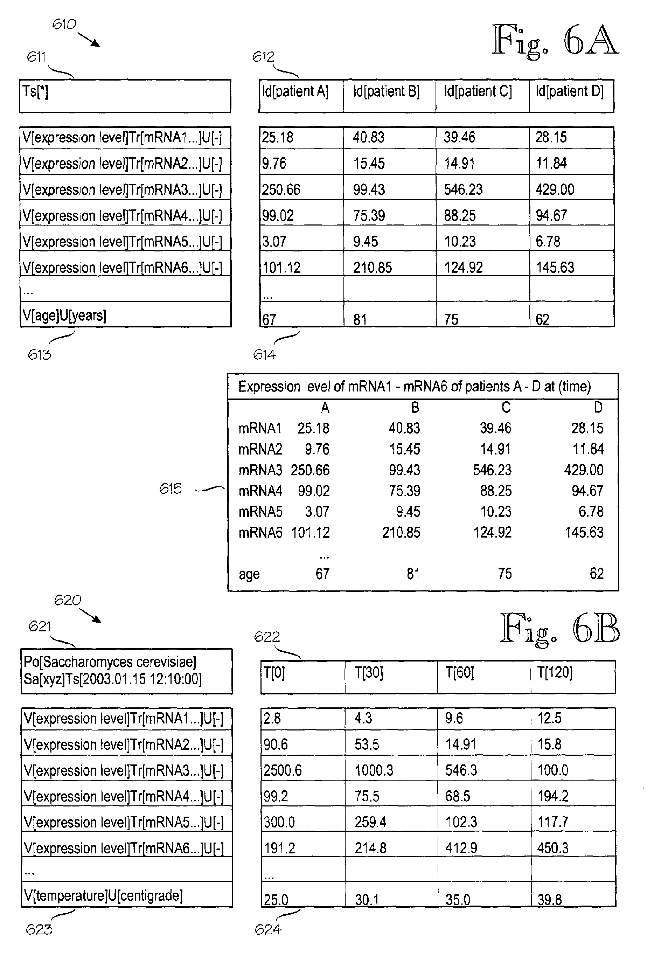

Note that there are many-to-many relationships from the base variables/units section 204 and the time section 206 to the data set section 202. This means that each data set 202 typically comprises one or more base variable/units and one or more time expressions. There is a many-to-many relationship between the data set section 202 and the experiments section 208, which means that each data set 202 relates one or more experiments 208, and each experiment relates to one or more data sets 202. A preferred implementation of the data sets section will be further described in connection with Figures 6A to 6C.

The base variables/units section 204 describes the base variables and units used in the IMS. In a simple implementation, each base variable record comprises unit field, which means that each base variable (eg mass) can be expressed in one unit only (eg kilograms). In a more flexible embodiment, the units are stored in a separate table, which permits expressing base variables in multiple units, such as kilograms or pounds.

Base variables are variables that can be used as such, or they can be combined to form more complex variables, such as the concentration of a compound in a specific sample at a specific point of time.

The time section 206 stores the time components of the data sets 202. Preferably, the time component of a data set comprises a relative (stopwatch) time and absolute (calendar) time. For example, the relative time can be used to describe the speed with which chemical reactions take place. There are also valid reasons for storing absolute time information along with each data set. The absolute time indicates when, in calendar time, the corresponding event took place. Such absolute time information can be used for calculating relative time between any experimental events. It can also be used for troubleshooting purposes. For example, if a faulty instrument is detected at a certain time, experiments made with that instrument prior to the detection of the fault should be checked.

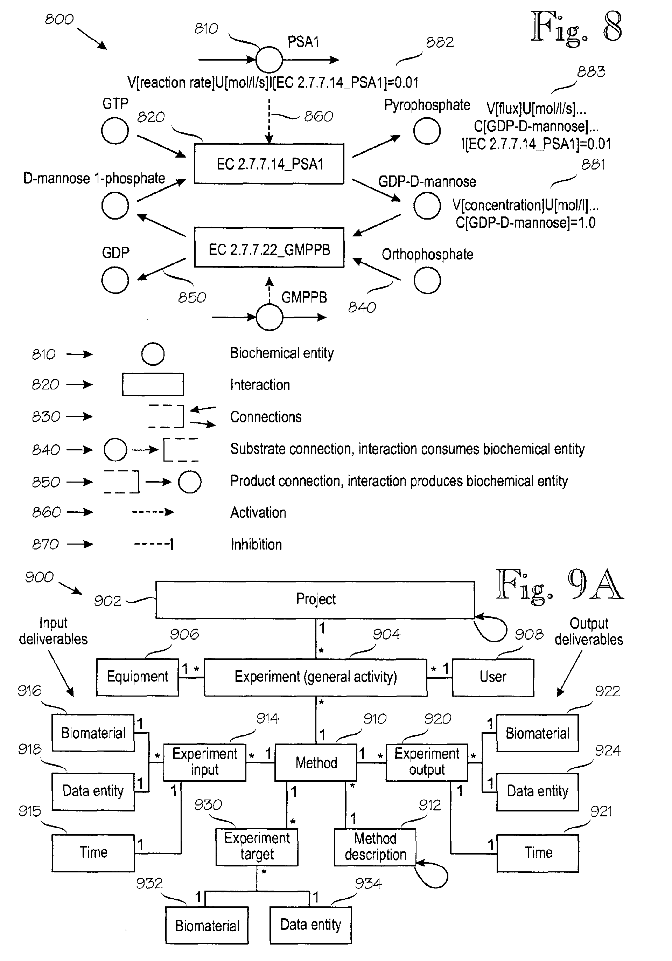

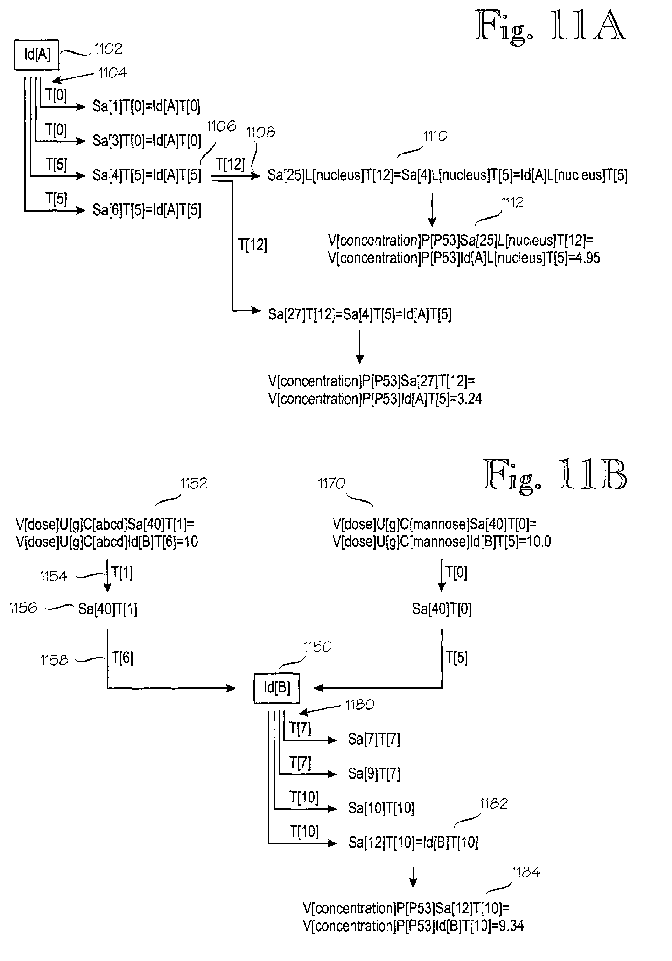

The experiments section 208 stores all experiments known to the IMS. There are two major experiment types, commonly called wet-lab and in-silico. But as seen from the point of view of the data sets 202, all experiments look the same. The experiments section 208 acts as a bridge between the data sets 202 and the two major experiment types. In addition to experiments already carried out, the experiments section 208 can be used to store future experiments. Preferred object-based implementations of experiments will be described in connection with Figure 9A. A key design goal of the experiments section is data traceability as will be further described in connection with Figure 11.

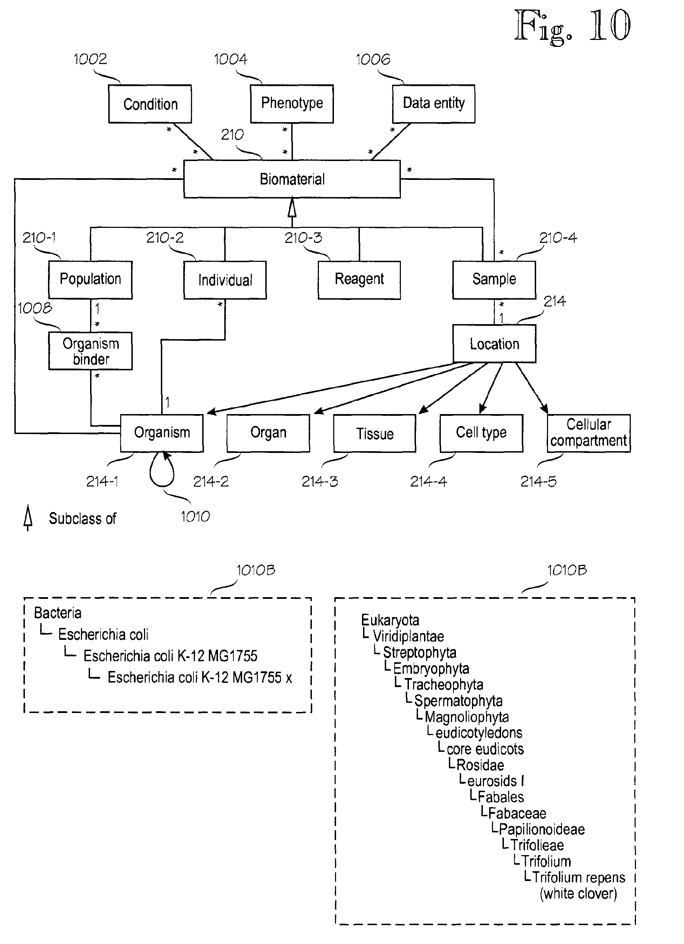

The biomaterial section 210 stores information about populations, individuals, reagents or samples of other biomaterials (anything that can be studied as a biochemical system or its component) in the IMS. Preferably, the biomaterials are described in data sets 202, by using the VDL to describe each biomaterial hierarchically, or in varying detail level, such as in terms of population, individual, reagent and sample. A preferred object-based implementation of the biomaterials section 210 will be described in connection with Figure 10.

While the biomaterial section 210 describes real-world biomaterials, the pathway section 212 describes theoretical models of biomaterials. Biochemical pathways are somewhat analogous to circuit diagrams of electronic circuits. There are several ways to describe pathways in an IMS, but Figure 2 outlines an advantageous implementation. In the example shown in Figure 2, each pathway 212 comprises one or more connections 216, each connection relating to one biochemical entity 218 and one interaction 222.

The biochemical entities are stored in a biochemical entity section 218. In the example shown in Figure 2, each biochemical entity is a class object whose subclasses are gene 218-1, transcript 218-2, protein 218-3, macromolecular complex 218-4 and compound 218-5. Preferably, there is an option to store abiotic stimuli 218 -6, such as temperature, having potential connections to interactions and potential effects to relevant kinetic laws.

A database reference section 220 acts as a bridge to external databases. Each database reference in section 220 is a relation between an internal biochemical entity 218 and an entity of an external database, such as a specific probe set of Affymetrix inc.

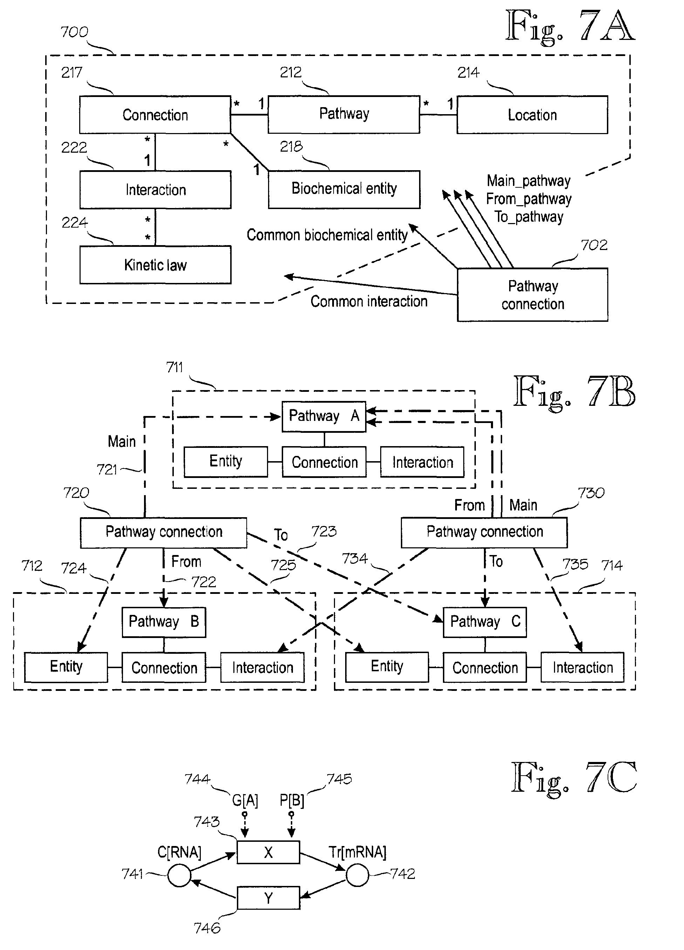

The interactions section 222 stores interactions, including reactions, between the various biochemical entities. The kinetic law section 224 describes kinetic laws (hypothetical or experimentally verified) that affect the interactions. Preferred and more detailed implementations of pathways will be described in connection with Figures 7A, 7B and 8.

According to a preferred embodiment of the invention, the IMS also stores multi-level location information 214. The multi-level location information is referenced by the biomaterial section 210 and the pathway section 212. For instance, as regards information relating to biomaterials, the organization shown in Figure 2 enables any level of detail or accuracy, from population level at one end down to spatial points (coordinates) within a cell at the other end. In the example shown in Figure 2, the location information comprises organism 214-1 (eg human), organ 214-2 (eg heart, stomach), tissue 214-3 (eg smooth muscle tissue, nervous tissue), cell type 214-4 (eg columnar epithelium cell), cellular compartment 214-5 (eg nucleus, cytoplasm) and spatial point 214-6 (eg x = 0.25, y = 0.50, z = 0.75 relative to the dimensions of a rectangular reference cell). The organism is preferably stored as a taxonomy tree that has a node to each known organism. The organ, tissue, cell type and cellular compartment blocks can be implemented as simple lists. A benefit of storing the location information as a reference to the predefined lists is that such referencing forces an automatic syntax check. Thus it is impossible to store a location information that references a non-existent or misspelled organ or organism.

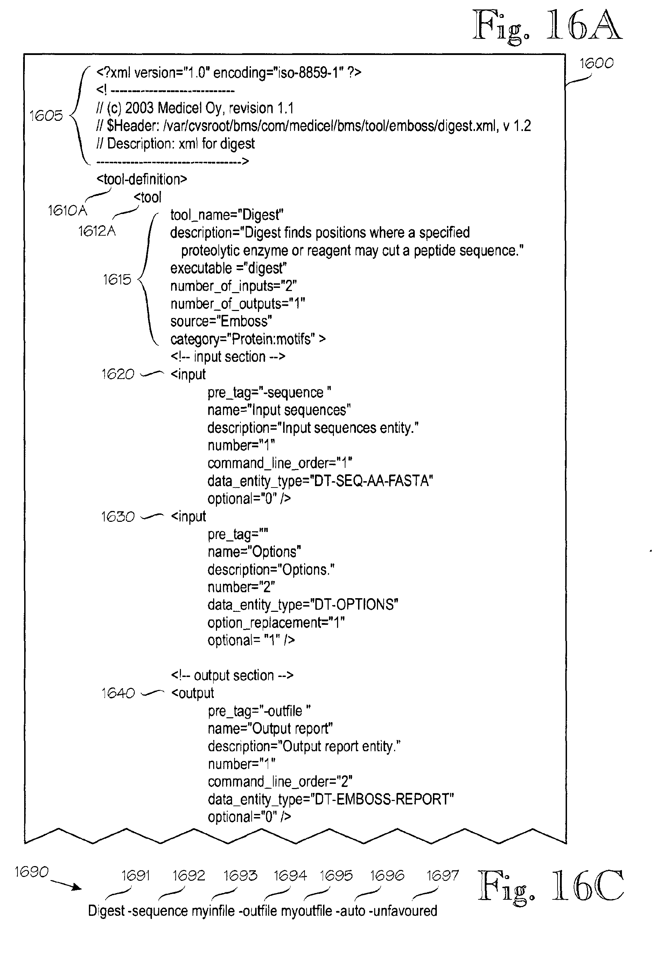

According to a further preferred embodiment of the invention, the location information can also comprise spatial information 214-6, such as a spatial point within the most detailed location in the organism-to-cell hierarchy. If the most detailed location indicates a specific cell or cellular compartment, the spatial point may further specify that information in terms of relative spatial coordinates. Depending on cell type, the spatial coordinates may be Cartesian or polar coordinates. Spatial points will be further discussed in connection with Figure 15.

In addition to the six levels of location hierarchy shown in Figure 2, it is advantageous to add some more relations to the organism. Relations particularly advantageous with the organism include, from specific to generic: individual, population and environment. With this arrangement of levels, a biochemical entity (such as a sample) can be associated to virtually any location on earth, with any desired resolution, down to a specific spatial coordinate within a cell.

A benefit of this kind of location information is an improved and systematic way to compare locations of samples and locations of theoretical constructs like pathways that need to be verified by relevant measurement results.

The multi-level location hierarchy shown in Figure 2 is particularly advantageous in connection with modern gene manipulation techniques, such as gene transfer and cloning. In comparison, some prior art systems label biological entities with simple text concatenations (such as "murine_P53"). Such a simple text concatenation hard-codes a specific organism to a specific location. If the location of the biological entity changes, its name changes as well, which disrupts the integrity of a well-defined database system. In contrast, the IMS as shown in Figure 2 can easily identify a pig's P53 gene transplanted to a mouse, for example, or make a distinction between a parent organism and a cloned one.

### Variable description language

Figures 3A to 3C illustrate a preferred variable description language, or "VDL". Generally speaking, a variable is anything that has a value and represents the state of a biochemical system (either a real-life biomaterial or a theoretical model). When an IMS is taken into use, the designer does not know what kinds of biomaterials will be encountered or what kinds of experiments will be carried out or what results are obtained from those experiments. Accordingly, variable descriptions have to be open to future extensions. On the other hand, openness and flexibility should not result in anarchy, which is why well-defined rules should be enforced on the variable descriptions. These needs are best served by an extendible variable description language ("VDL").

extendible markup language (XML) is one example of an extendible language that could, in principle, be used to describe biochemical variables. XML expressions are rather easily interpretable by computers. However, XML expressions tend to be very long, which makes them poorly readable to humans. Accordingly, there is a need for an extendible VDL that is more compact and more easily readable to humans and computers than XML is.

The idea of an extendible VDL is that the allowable variable expressions are "free but not chaotic". To put this idea more formally, we can say that the IMS should only permit predetermined variables but the set of predetermined variables should be extendible without programming skills. For example, if a syntax check to be performed on the variable expressions is firmly coded in a syntax check routine, any new variable expression requires reprogramming. An optimal compromise between rigid order and chaos can be implemented by storing permissible variable keywords in a data structure, such as a data table or file, that is modifiable without programming. Normal access grant techniques can be employed to determine which users are authorized to add new permissible variable keywords.

Figure 3A illustrates a variable description in a preferred VDL. A variable description 30 comprises one or more pairs 31 of a keyword and name, separated by delimiters. As shown in the example of Figure 3A, each keyword-name pair 31 consists of a keyword 32, an opening delimiter (such as an opening bracket) 33, a (variable) name 34 and a closing delimiter (such as a closing bracket) 35. For example, "Ts[2002-11-26 18:00:00]" (without the quotes) is an example of a time stamp. If there are multiple keyword-name pairs 31, the pairs can be separated by a separator 36, such as a space character or a suitable preposition. The separator and the second keyword-name pair 31 are drawn with dashed lines because they are optional. The ampersands between the elements 32 to 36 denote string concatenation. That is, the ampersands are not included in a variable description.

As regards the syntax of the language, a variable description may comprise an arbitrary number of keyword-name pairs 31. But an arbitrary combination of pairs 31, such as a concentration of time, may not be semantically meaningful.

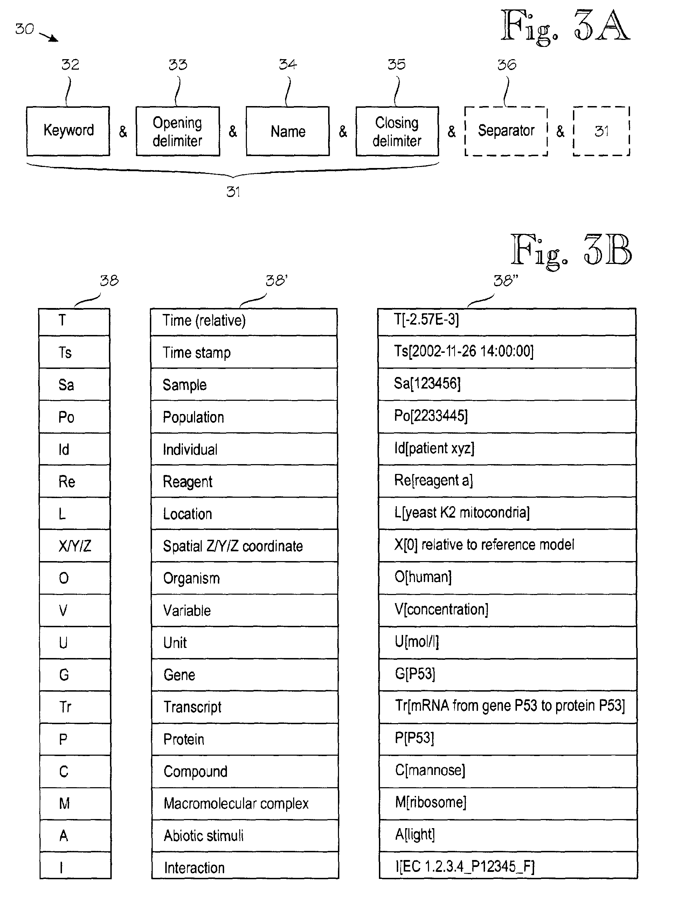

Figure 3B shows a table 38 of typical keywords. Next to each entry in table 38 is its plaintext description 38' and an illustrative example 38". Note that the table 38 is stored in the IMS but the remaining tables 38' and 38" are not necessarily stored (they are only intended to clarify the meaning of each keyword in table 38). For example the example for keyword "T" is "T[-2.57E-3]" which is one way of expressing minus 2.57 milliseconds prior to a time reference. The time reference may be indicated by a timestamp keyword "Ts".

The T and Ts keywords implement the relative (stopwatch) time and absolute (calendar) time, respectively. A slight disadvantage of expressing time as a combination of relative and absolute time is that each point of time has a theoretically infinite set of equivalent expressions. For example, "Ts[2002-11-26 18:00:30]" and "Ts[2002-11-26 18:00:00]T[00:00:30]" are equivalent. Accordingly, there is preferably a search logic that processes the expressions of time in a meaningful manner.

By storing an entry for each permissible keyword in the table 38 within the IMS, it is possible to force an automatic syntax check on variables to be entered, as will be shown in Figure 3C.

The syntax of the preferred VDL may be formally expressed as follows:

<variable description>::=<keyword>"["<name>"]"{{separator}<keyword>"["<name>"]"}<end>

<keyword>::=<one of predetermined keywords, see eg table 38>

<name>::=<character string> | "*" for any name in a relevant data table

The purpose of explicit delimiters, such as "[" and "]" around the name is to permit any characters within the name, including spaces (but excluding the delimiters, of course).

A preferred set of keywords 38 comprises three kinds of keywords: what, where and when. The "what" keywords, such as variable, unit, biochemical entity, interaction, etc., indicate what was or will be observed. The "where" keywords, such as sample, population, individual, location, etc., indicate where the observation was or will be made. The "when" keywords, such as time or time stamp, indicate the time of the observation.

Figure 3C illustrates an optional process for automatic syntax checking. A benefit of a formal VDL is that it permits an automatic syntax check. Figure 3C illustrates a state machine 300 for performing such a syntax check. State machines can be implemented as computer routines. From an initial state 302 a valid keyword causes a transition to a first intermediate state 304. Anything else causes a transition to an error state 312. From the first intermediate state 304, an opening delimiter causes a transition to a second intermediate state 306. Anything else causes a transition to the error state 312.

After the opening delimiter, any characters except a closing delimiter are accepted as parts of the name, and the state machine remains in the second intermediate state 306. Only a premature ending of the variable expression causes a transition to an error state 312. A closing delimiter causes a transition to a third intermediate state 308, in which one keyword/name pair has been validly detected. A valid separator character causes a return to the first intermediate state 304. Detecting the end of the variable expression causes a transition to "OK" state 310 in which the variable expression is deemed syntactically correct.

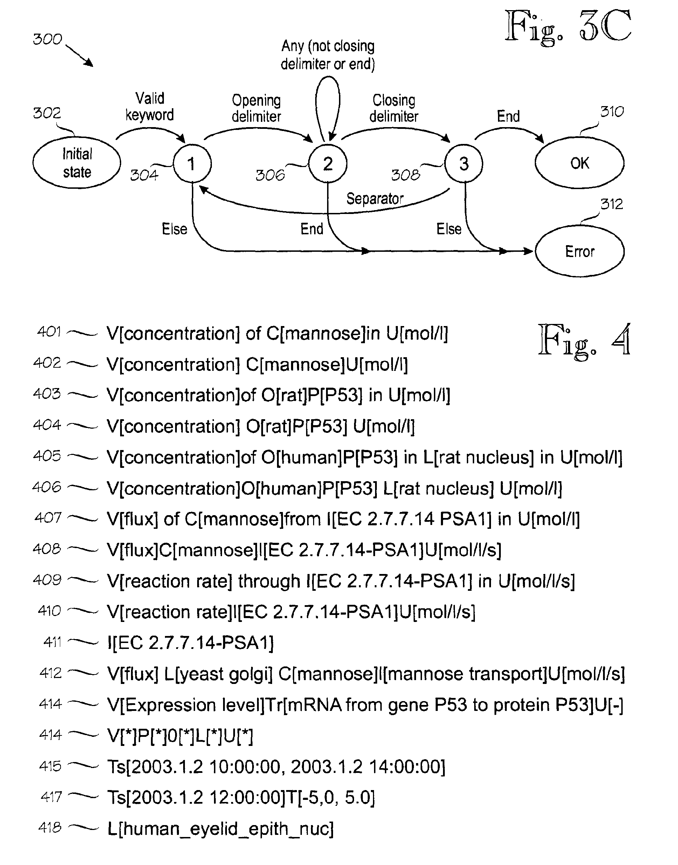

Figure 4 shows examples of compound variable expressions in the VDL. Compound variable expressions are expressions with multiple keyword/name pairs. Note how variables get more specific when qualifiers are added. Reference signs 401 to 410 denote five pairs of equivalent expressions such that the first expression of each pair is longer or more verbose and the second is more compact. For a computer, the verbose and compact expressions are equal, but human readers may find the verbose form easier to understand. By referencing table 38, the expressions in Figure 4 are self-explanatory. For example, expressions 409 and 410 define reaction rate through interaction EC 2.7.7.13-PSA1 in moles per litre per second. Reference sign 414 denotes variable expression "V[*]P[*]0[*]U[*]" which means any variable of any protein of any organism in any units. Reference signs 415 and 416 denote two different variable expression for two different expressions of time. Variable expression 415 defines a three-hour time interval and variable expression 417 defines a 10-second time interval (beginning five seconds before and ending five seconds after the timestamp). Variable expression 418 is an expression of a hierarchical location expression. As shown in Figure 2, the location information is preferably hierarchical and comprises database relations to organism 214-1, organ 214-2, tissue 214-3, cell type 214-4, cellular compartment 214-5 and/or spatial point 214-6, as appropriate. Variable expression 418 ("L[human_eyelid_epith_nuc]") is a visualized expression of such a multi-level hierarchical location information. Its organism relation 214-1 indicates a human, its organ relation 214-2 indicates eyelid, its cell type relation 214-4 indicates epithelial cell and its cellular compartment relation 214-5 indicates cell nucleus. In this example, the multi-level hierarchical location does not indicate any specific tissue or spatial point within the cell or cellular compartment.

Note that regardless of the language of humans using the IMS, it is beneficial to agree on one language for the variable expressions. Alternatively, the IMS may comprise a translation system to translate the variable expressions to various human languages.

The VDL substantially as described above is well-defined because only expressions that pass the syntax check shown in Figure 3C are accepted. The VDL is open because the permissible keywords are stored in table 38 which is extendible. The VDL is compact because substantially the minimum number of letters or characters are used for the keywords. The most common keywords are comprised of a single letter, or two letters if a one-letter keyword is ambiguous. Another reason for the compactness of the VDL described herein is that it does not use keywords in pairs of opening keyword - closing keyword, such as "<ListOfProteins> ... </ListOfProteins>", which is typical of XML and its variants. Yet another characteristic feature of the VDL described herein is that the keywords are not separated by paragraph (new line) characters, which is why most expressions require much less than a single line in a document or on a computer display. Actually, the inventive VDL does not require any separator characters (only closing delimiters, such as "]"), but separator characters, such as spaces or prepositions, may be used to enhance readability to humans.

**Data contexts**

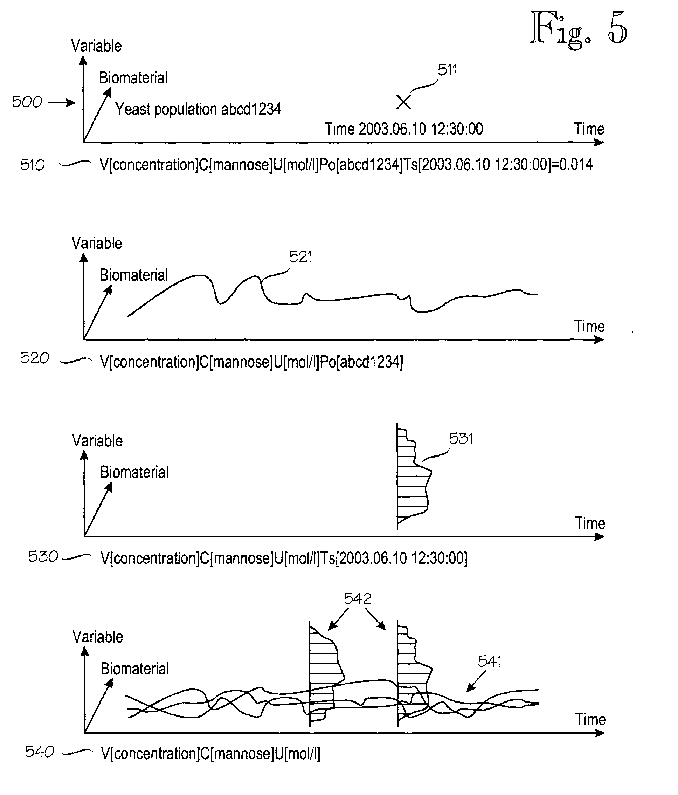

Figure 5 shows how the VDL can be used to express different data contexts or scopes of biochemical research. All variables, whether sampled, measured, modelled, simulated or processed in any manner, can be expressed as:

a) single values for a biomaterial sample at a point of time;
b) functions of time for the biomaterial;
c) stochastic variables with their distributions at each point of time based on available biomaterial samples; or
d) stochastic processes in the biochemical data context.

a), b) and c) are projections of d) which is the richest representation of the system. All data in the IMS exists in a three-dimensional context space that has relations to:

1. list of variables ("what");
2. list of real-life biomaterials or pathway models ("where");
3. list of time points or time intervals ("when").

Reference numeral 500 generally denotes the N + 2 dimensional context space having one axis for each of variables (N), biomaterials and time. A very detailed variable expression 510 specifies a variable (concentration of mannose in moles/l), biomaterial (population abcd1234) and a timestamp (10 June 2003 at 12:30). The value of the variable is 1.3 moles/l. Since the variable expression 510 specifies all the coordinates in the context space, it is represented by a point 511 in the context space 500.

The next variable expression 520 is less detailed in that it does not specify time. Accordingly, the variable expression 520 is represented by a function 521 of time in the context space 500.

The third variable expression 530 does specify time but not biomaterial. Accordingly, it is represented by a distribution 531 of all biomaterials belonging to the experiment at the specified time.

The fourth variable expression 540 specifies neither time nor biomaterial. It is represented by a set 541 of functions of time and a set 542 of distributions for the various biomaterials.

By means of the various expressions made possible by the variable description language and suitably-organized data sets (to be described next), researchers have virtually unlimited possibilities to study the time-state space of a biochemical system as a multidimensional stochastic process. The probabilistic aspects of the system are based on the event space of relevant biomaterials, and the dynamic aspects are based on the time-space. Biomaterial data and time can be registered when the relevant experiments are documented.

All quantitative measurements, data analyses, models and simulation results can be reused in new analysis techniques to find relevant background information, such as phenotypes of measured biomaterials when the data needs to be interpreted for various applications.

**Data sets**

Figures 6A to 6C illustrate data sets according to various preferred embodiments of the invention. Both wet-lab and in-silico experiment types are preferably stored as data sets of similar construction. By storing data related to wet-lab and in-silico experiments in similarly constructed data sets, it is possible to use output data from a wet-lab experiment as input data to an in-silico experiment, for example, without any intervening data format conversions. In Figure 6A, an exemplary data set 610 describes expression levels of a number of mRNA molecules (mRNA1 through mRNA6 are shown). Data set 610 is an example of a data set stored in the data set section 202 shown in Figure 2. The data set 610 comprises four matrixes 611 through 614. A variable value matrix 614 describes the values of the variables values in a row-column organization. A row description list 613 specifies the meaning of the rows of the variable value matrix. A column description list 612 specifies the meaning of the columns of the variable value matrix. Finally, a fixed dimension description 611 specifies one or more fixed dimensions that are common to all values in the variable value matrix 614. Note that the variable value matrix 614 is comprised of scalar numbers. The remaining matrixes 610 to 613 use the VDL to specify the meaning of their contents.

Figure 6A also shows a human-readable version 615 of the data set 610. Note that the human-readable version 615 of the data set is only shown for better understanding of this embodiment. The human-readable version 615 is not necessarily stored anywhere, and can be created from the data set 610 automatically whenever a need to do so arises. The human-readable version 615 is an example of data sets, such as spreadsheet files, that are typically stored in prior art IMS systems for biochemical research. The IMS preferably contains a user interface logic for automatic two-way conversion between the storage format 611 - 614 and the human-readable version 615.

Figure 6B shows another data set 620. The data set 620 also specifies expression levels of six mRNA molecules, but these are not expression levels of different individuals but of a single population at four different times. In the data set 620, the fixed dimension description 621 specifies that the data relates to sample xyz of a certain yeast at a certain date and time. The column description list 622 specifies that the columns specify data for four instances of time, namely 0, 30, 60 and 120 seconds after the time stamp in the fixed dimension description 621. The row description list 623 is very similar to the corresponding list 613 in the previous example, the only difference being that the last row indicates temperature instead of patient's age. The variable value matrix 624 contains the actual numerical values.

The division of each data set (eg data set 610) to four different components (the matrixes 611 to 614) can be implemented so that each matrix 611 to 614 is a separately addressable data structure, such as a file in the computer's file system. Alternatively, the variable value matrix can be stored in a single addressable data structure, while the remaining three matrixes (the fixed dimension description and the row/column descriptors) can be stored in a second data structure, such as a single file with headings "common", "rows" and "column". A key element here is the fact that the variable value matrix is stored in a separate data structure because it is the component of the data set that holds the actual numerical values. If the numerical values are stored in a separately addressable data structure, such as a file or table, it can be easily processed by various data processing applications, such as data mining or the like. Another benefit is that the individual data elements that make up the various matrixes need not be processed by SQL queries. An SQL query only retrieves an address or other identifier of a data set but not the individual data elements, such as the numbers and descriptions within the matrixes 611 to 614.

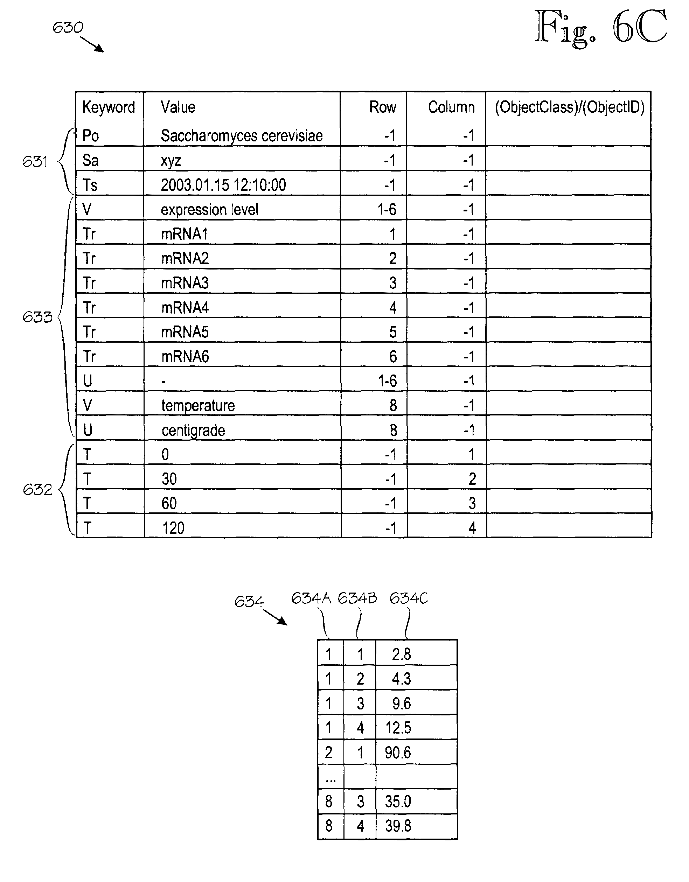

Figure 6C shows an alternate implementation of the data sets. This implementation is particularly advantageous with sparse data or if there are redundant variable descriptions that can be stored efficiently by storing each data item only once in an appropriate data table. The example shown in Figure 6C stores precisely the same data that was shown in Figure 6B, but in a different organization. A variable value matrix 634 is a 3*n matrix, wherein n is the number of actual data items. The data items are stored in column 634C, which comprises precisely the same data as the variable value matrix 622 of Figure 6B (although some elements are hidden, as indicated by the ellipsis). In addition to column 634C, the variable value matrix 634 comprises a row indicator column 634A and a column indicator column 634B, which indicate the row and column which the corresponding data item belongs to. The variable value matrix 634 is particularly advantageous when data is very sparse, because null entries need not be stored. On the other hand, the variable value matrix 634 requires explicit row and column indicators.

In the example of Figure 6C, the significance of the data, ie, the row/column descriptors and the common descriptors are stored in a matrix or table 630, that has entries for keyword, value, row and column. Section 631 of the matrix 630 corresponds to the fixed dimension description 621 shown in Figure 6B. The three elements in the fixed dimension description 621, ie, population, sample and time stamp, are stored as separate rows in section 631 of matrix 630. For instance, the first row has an entry of "Po" (=population) for the keyword, "Saccharomyces cerevisiae" for the corresponding value, and "-1" for each of the row and column. In this example, "-1" is a special value which is valid for all rows or column. As the section 631 is valid for all rows and columns, its contents correspond to the fixed dimension description 621 shown in Figure 6B. Section 633 corresponds to the row description 623 of Figure 6B. In section 633, the column indicators are "-1", which means "any column". The first line of section 633 means that the keyword "V" (=variable) and its value ("expression level") are valid for rows 1 to 6. The next six lines are six different row descriptors for rows 1 to 6, and so on. Finally, section 632 correspond to the column description 622 in Figure 6B. Here, the rows are all "-1", since the column descriptors are valid for all rows.

The matrixes 630 and 634 shown in Figure 6C comprise precisely the same information as the common and row/column descriptors 621 to 623 in Figure 6B, as far as human readers are concerned. But interpretation of data by computers can be facilitated by storing separate entries for object class and object identifier. This feature eliminates some extra processing steps, such as data look-up via a keyword table 38 shown in Figure 3B.

**Pathways**

Figure 7A is a block diagram of a pathway as stored in the IMS. An IMS according to a preferred embodiment of the invention describes each biochemical system by means of a structured pathway model 700 of system components and inter-component connections. The system components are biochemical entities 218 and interactions 222. The connections 216 between the biochemical entities 218 and interactions 222 are recognized as independent objects representing the role (eg substrate, product, activator or inhibitor) of each biochemical entity in each interaction for each pathway. A connection can hold attributes that are specific to each biochemical entity and interaction pair (such as a stoichiometric coefficient). As stated earlier, the IMS preferably stores location information, and each pathway 212 relates to a biological location 214. One biological location might be described by one or more pathways depending on the level of details that have been included into a pathway.

As shown in Figure 7A, each connection 216 acts as a T joint that joins three elements, namely an interaction 222, a biochemical entity 218 and a pathway 212. In other words, the join of an interaction 222 and a biochemical entity 218 is pathway-specific, as opposed to global. This means that a biochemical researcher can change the interaction data relating to a given biochemical entity, and the change only affects the specific pathway indicated by the pathway element 212. This feature is believed to lower the psychological threshold faced by researchers to make changes to a pathway definition.

In an object-based implementation, the biochemical pathway model is based on three categories of objects: biochemical entities (molecules) 218, interactions (chemical reactions, transcription, translation, assembly, disassembly, translocation, etc) 222, and connections 216 between the biochemical entities and interactions for a pathway. The idea is to separate these three objects in order to use them with their own attributes and to use the connection to hold the role (such as substrate, product, activator or inhibitor) and stoichiometric coefficients of each biochemical entity in each interaction that takes place in a particular biochemical network. A benefit of this approach is the clarity of the explicit model and easy synchronization when several users are modifying the same pathway connection by connection. The user interface logic can be designed to provide easily understandable visualizations of the pathways, as will be shown in connection with Figure 8.

The kinetic law section 224 describes theoretical or experimental kinetic laws that affect the interactions. For example, a flux from a substrate to a chemical reaction can be expressed by the following formula:

$$V = \frac{V\,max \cdot [S] \cdot [E]}{K + [S]}$$

wherein V is the flux rate of the substrate, Vmax and K are constants, [S] is the substrate concentration and [E] is the enzyme concentration. The reaction rate through the interaction can be calculated by dividing the flux by the stoichiometric coefficient of the substrate. Conversely, each kinetic law represents the reaction rate of an interaction, whereby any particular flux can be calculated by multiplying the reaction rate by the stoichiometric coefficients of the particular connections. The above kinetic law as the reaction rate of interaction EC2.7.7.14_PSA1 in Figure 8 can be expressed in VDL as follows:

$$V[rate]I[EC2.7.7.14\_PSA1] = Vmax \cdot V[concentration]C[GTP] \cdot V[concentration]P[PSA1]/(K +$$

$$V[concentration]C[GTP])$$

The flux from interaction EC2.7.7.14_PSA1 to compound GDP-D-mannose can be expressed in VDL as follows:

$$V[flux]I[EC2.7.7.14\_PSA1]C[GDP\text{-}D\text{-}mannose] =$$

$$c1 \cdot V[rate]I[EC2.7.7.14\_PSA1] = Vmax \cdot V[concentration]C[GTP] \cdot V(concentration]P[PSA1]/(K$$

$$+ V[concentration]C[GTP]),$$

where c1 is the stoichiometric coefficient of the connection from interaction EC2.7.7.14_PSA1 to compound GDP-D-mannose and c1 = 1.

In the above example, the kinetic law is a continuous function of variables V[concentration]C[GTP] and V[concentration] P[PSA1]. In addition, a proper description of some pathways requires discontinuous kinetic laws.

Figure 7C shows a visualized form of a hybrid pathway model that comprises both analogue (continuous) and Boolean (discrete) equations. In this model, compound RNA 741 is converted to transcript mRNA 742 via interaction (reaction) X 743 but only if gene A 744 and protein B 745 are present. Interaction Y 746 is the inverse process of interaction X 743 and transforms transcript mRNA back to compound RNA.

The kinetic law as the reaction rate of interaction X in Figure 7C can be expressed as a discontinuous Boolean function of VDL conditions as follows:

$$V[rate]I[X] =$$

$$k \text{ IF } V[count]G[A] > 0 \text{ AND } V[count]P[B] > 0 \text{ and } V[count]C[RNA] > 0 \text{ ELSE } 0$$

The flux from interaction X to transcript mRNA can be expressed in VDL as follows:

$$V[flux]I[X]Tr[mRNA]=$$

$$c2 \cdot V[rate]I[X] =$$

$$k \text{ IF } V[count]G[A] > 0 \text{ AND } V[count]P[B] > 0 \text{ and } V[count]C[RNA] > 0 \text{ ELSE } 0$$

where c2 is the stoichiometric coefficient of the connection from interaction X to transcript mRNA and c2 = 1.
Let the flux from interaction Y to compound RNA in Figure 7C be a continuous function of the count of transcript mRNA as follows:

$$V[flux]I[Y]C[RNA] =$$

$$c3 \cdot V[rate]I[Y] = c3 \cdot k2 \cdot V[count]Tr[mRNA]$$

where c3 is the stoichiometric coefficient of the connection from interaction X to transcript mRNA and k2 is another constant of this kinetic law.
Each variable represented in the kinetic laws may be specified with a particular location L[...] if the concentration or count of a biochemical entity depends on a particular location.

A biochemical network may not be valid everywhere. In other words, the network is typically location-dependent. That is why there are relations between pathways 212 and biologically relevant discrete locations 214, as shown in Figures 1 and 7A.

A complex pathway can contain other pathways 700. In order to connect different pathways 700 together, the model supports pathway connections 702, each of which has up to five relations which will be described in connection with Figure 7B.

Figure 7B shows an example of complex pathway that contains simpler pathways. Two or more pathways can be combined if they have common biochemical entities that can move as such between relevant locations or common interactions (eg translocation type interaction that moves biochemical entities from one location to another). Otherwise, the pathways are considered isolated.

Pathway A, denoted by reference sign 711, is a main pathway to pathways B and C, denoted by reference signs 712 and 713, respectively. The pathways 711 to 713 are basically similar to the pathway 700 described above. There are two pathway connections 720 and 730 that couple the pathways B and C, 712 and 713, to the main pathway A, 711. For instance, pathway connection 720 has a main-pathway relation 721 to pathway A, 711; a from-pathway relation 722 to pathway B, 712; and a to-pathway relation 723 to pathway C, 713. In addition, it has common-entity relations 724, 725 to pathways B 712 and C 713. In plain language, the common-entity relations 724, 725 mean that pathways B and C share the biological entity indicated by the relations 724, 725.

The other pathway connection 730 has both main-pathway and from-pathway relations to pathway A 711, and a to-pathway relation to pathway C, 713. In addition, it has common-interaction relations 734, 735 to pathways B, 712 and C, 713. This means that pathways B and C share the interaction indicated by the relations 734, 735.

The pathway model described above supports incomplete pathway models that can be built gradually, along with increasing knowledge. Researchers can select detail levels as needed. Some pathways may be described in a relatively coarse manner. Other pathways may be described down to kinetic laws and/or spatial coordinates. The model also supports incomplete information from existing gene sequence databases. For example, some pathway descriptions may describe gene transcription and translation separately, while other treat them as one combined interaction. Each amino acid may be treated separately or all amino acids may be combined to one entity called amino adds.

The pathway model also supports automatic modelling processes. Node equations can be generated automatically for time derivatives of concentrations of each biochemical entity when relevant kinetic laws are available for each interaction. As a special case, stoichiometric balance equations can be automatically generated for flux balance analyses. The pathway model also supports automatic end-to-end workflows, including extraction of measurement data via modelling, inclusion of additional constrains and solving of equation groups, up to various data analyses and potential automatic annotations.

Automatic pathway modelling can be based on pathway topology data, the VDL expressions that are used to describe variable names, the applicable kinetic laws and mathematical or logical operators and functions. Parameters not known precisely can be estimated or inferred from the measurement data. Default units can be used in order to simplify variable description language expressions.

If the kinetic laws are continuous functions of VDL variables, the quantitative variables (eg concentration) of biochemical entities can be modelled as ordinary differential equations of these quantitative variables. The ordinary differential equations are formed by setting a time derivative of the quantitative variable of each biochemical entity equal to the sum of fluxes coming from all interactions connected to the biochemical entity and subtracting all the outgoing fluxes from the biochemical entity to all interactions connected to the biochemical entity.

Example:

$$dV[concentration]C[GDP\text{-}D\text{-}mannose]/dV[time] =$$
$$V[flux]I[EC\ 2.7.7.13\_PSA1]C[GDP\text{-}D\text{-}mannose] + \ldots$$
$$-\ V[flux]C[GDP\text{-}D\text{-}mannose]I[\ EC\ \ldots\qquad]-\ldots$$

$$\ldots$$

$$dV[concentration\ ]C[water]/dV[time] = V[flux]C[water]I[EC\ \ldots\qquad] + \ldots$$
$$-\ V[flux]C[water]I[\ EC\ \ldots\qquad]-\ldots$$

On the other hand, if the kinetic laws are discontinuous functions of VDL variables, the quantitative variables (eg concentration or count) of biochemical entities can be modelled as difference equations of these quantitative variables. The difference equations are formed by setting the difference of the quantitative variable of each biochemical entity in two time points equal to the sum of the incoming quantities from all interactions connected to the biochemical entity and subtracting all the outgoing quantities from the biochemical entity to all interactions connected to the biochemical entity in the time interval between the time points of the difference.

Example:

$$V[count]Tr[mRNA]T[t+\Delta t] - V[count]Tr[mRNA]T[t] =$$

$$V[flux]I[X]Tr[mRNA]\bullet \Delta t - V[flux]I[Y]Tr[mRNA] \bullet \Delta t + V[...]... - V[....]...$$

$$V[count]C[RNA]T[t+\Delta t] - V[count]C[RNA]T[t]=$$

$$V[flux]I[Y]C[RNA]\bullet \Delta t -V[flux]I[X]C[RNA]\bullet \Delta t+ V[...]... - V[....]...$$

...

If there are both continuous and discontinuous kinetic laws associated with an interaction that connects a biochemical entity, a difference equation is written from the biochemical entity such that continuous or discontinuous fluxes are added or subtracted depending on the direction of each connection.

In this way a complete "hybrid" equation system can be generated for simulation purposes with given initial or boundary conditions. Initial conditions and boundary conditions can be represented by the data sets described above (see Figures 6A to 6C).

In the differential and difference equations described above, the biochemical entity-specific fluxes can be replaced by reaction rates multiplied by stoichiometric coefficients.

In a static case, the derivatives and differences are zeros. This leads to a flux balance model with a set of algebraic equations of reaction rate variables (kinetic laws are not needed), wherein the set of algebraic equations describe the feasible set of the reaction rates of specific interactions.

$$0 = V[rate]I[EC\ 2.7.7.13\_PSA1] + ...$$

$$- V[rate]I[\ EC\ ... \qquad ] - ...$$

$$...$$

$$0 = V[rate]I[EC\ ... \qquad ] + ...$$

$$- V[rate]I[\ EC\ ... \qquad ] - ...$$

**or**

$$0 = V[rate]I[X] - V[rate]I[Y] + V[...]... - V[...]....$$

$$0 = V[rate]I[Y] -V[rate]I[X] + V[...]... - V[...]...$$

$$...$$

Users can provide their objective functions and additional constraints or measurement results that limit the feasible set of solutions.

Yet another preferred feature is the capability to model noise in a flux-balance analysis. We can add artificial noise variables that need to be minimized in the objective function. The noise variables are given in the data sets described above. This helps to tolerate inaccurate measurements with reasonable results.

The model described herein also supports visualization of pathway solutions (active constraints). A general case, the modelling leads to a hybrid equations model where kinetic laws are needed. They can be accumulated in the database in different ways but there may be some default laws that can be used as needed. In general equations, interaction-specific reaction rates are replaced by kinetic laws, such as Michaels-Menten laws, that contain concentrations of enzymes and substrates. Example:

V[reaction rate]I[EC 2.7.7.13_PSA1] =

5.2*V[concentration]P[PSA1]*V[concentration]C[...] / ( 3.4 + V[concentration]C[...] )

The equations can be converted to the form:

dV[concentration ]C[GDP-D-mannose]/dV[time] =

5.2*V[concentration]P[PSA1]*V[concentration]C[...] / ( 3.4 +

V[concentration]C[...] ) + ...

-7.9*V[concentration]P[ ... ]*V[concentration]C[...]/ ( ... )


...

dV[concentration ]C[water]/dV[time] = 10.0*V[concentration] P[...]*V[concentration]C[...] /(...) + ...

- 8.6*V[concentration ]P[...]*V[concentration]C[...] /(...) - ...

or


V[count]Tr[mRNA]T[t+$\Delta$t] - V[count]Tr[mRNA]T[t] =

(k IF V[count]G[A] > 0 AND V[count]P[B] > 0 and V[count]C[RNA] > 0 ELSE 0) •$\Delta$t

– c3• k2• V[count]Tr[mRNA] • $\Delta$t + V[...]... – V[....]...

V[count]C[RNA]T[t+$\Delta$t] - V[count]C[RNA]T[t]=

c3• k2• V[count]Tr[mRNA]• $\Delta$t -(k IF V[count]G[A] > 0 AND V[count]P[B] > 0 and V[count]C[RNA] > 0 ELSE 0) •$\Delta$t + V[...]... – V[....]...

There are alternative implementations. For example, instead of the substitution made above, we can calculate kinetic laws separately and substitute the numeric values to specific reaction rates iteratively.

A benefit of such a structured pathway model, wherein the pathway elements are associated with interaction data, such as interaction type and/or stoichiometric coefficients and/or location, is that flux rate equations, such as the equations described above, can be generated by an automatic modelling process, which greatly facilitates computer-aided simulation of biochemical pathways. Because each kinetic law has a database relation to an interaction and each interaction relates, via a specific connection, to a biochemical entity, the modelling process can automatically combine all kinetic laws that describe the creation or consumption of a specific biochemical entity and thereby automatically generate flux-balance equations according to the above-described examples.

Another benefit of such a structured pathway model is that hierarchical pathways can be interpreted by computers. For instance, the user interface logic may be able to provide easily understandable visualizations of the hierarchical pathways as will be shown in connection with Figure 8.

Figure 8 shows a visualized form of a pathway, generally denoted by reference numeral 800. A user interface logic draws the visualized pathway 800 based on the elements 212 to 224 shown in Figures 1 and 7A. Circles 810 represent biochemical entities. Boxes 820 represent interactions and edges 830 represent connections. Solid arrows 840 from a biochemical entity to an interaction represent substrate connections where the biochemical entity is consumed by the interaction. Solid arrows 850 from an interaction to a biochemical entity represent product connection where the biochemical entity is produced by the interaction. Dashed arrows 860 represent activations where the biochemical entity is neither consumed nor produced but it enables or accelerates the interaction. Dashed lines with bar terminals 870 represent inhibitions where the biochemical entity is neither consumed nor produced but it inhibits or slows down the interaction. The non-zero stoichiometric coefficients are associated with the substrate or product connections 840, 850. In control connections (eg activation 860 or inhibition 870) the stoichiometric coefficients are zero.

Also, measured or controlled variables can be visualized and localized on relevant biochemical entities. For example, reference numeral 881 denotes the concentration of a biochemical entity, reference numeral 882 denotes the reaction rate of an interaction and reference numeral 883 denotes the flux of a connection.

The precise roles of connections, kinetic laws associated with interactions and the biologically relevant location of each pathway provide improvements over prior art pathway models. For instance, a model as shown in Figures 7A to 8 supports descriptions of varying detail levels by varying the number of elements. Further, the model supports the inclusion of explicit kinetic laws if they are known.

This technique supports graphical representations of measurement results on displayed pathways as well. The measured variables can be correlated to the details of a graphical pathway representation based on the names of the objects.

Note that the data base structure denoted by reference numerals 200 and 700 (Figures 2 and 7A) provide a means for storing the topology of a biochemical pathway but not its visualization 800. The visualization can be generated from the topology, and stored later, as follows. The elements and interconnections of the visualization 800 are directly based in the stored pathways 700. The locations of the displayed elements can be initially selected by a software routine that optimizes some predetermined criterion, such as the number of overlapping connections. Such techniques are known from the field of printed-circuit design. The IMS may provide the user with graphical tools for manually cleaning up the visualization. The placement of each element in the manually-edited version may then be stored in a separate data structure, such as a file.

**Experiments**

The IMS preferably comprises an experiment project manager. A project comprises one or more experiments, such as sampling, treatment, perturbation, feeding, cultivation, manipulation, purification, cloning or other combining, separation, measurement, classification, documentation, or in-silico workflows.

A benefit of an experiment project manager is that all the measurement results or controlled conditions or perturbations ("what"), biomaterials and locations in biomaterials ("where") and timing of relevant experiments ("when") and methods ("how") can be registered for the interpretation of the experiment data. Another benefit comes from the possibility to utilize the variable description language when storing experiment data as data sets explained earlier.

Figure 9A shows an experiment object in an experiments section of the IMS. As stored in the IMS, each project 902 comprises one or more experiments 904. Each experiment 904 has relations to equipment data 906, user data 908 and method data 910. Each method entity 910 relates to experiment input 914 and experiment output 920. The experiment input 914 connects relevant input, such as a biomaterial 916 (eg population, individual, reagent or sample) or a data entity 918 (eg controlled conditions) to the experiment, along with relevant time information.

The experiment output 920 connects relevant output, such as a biomaterial 922 (eg population, individual, reagent or sample) or a data entity 924 (eg measurement results, documents, classification results or other results) to the experiment, along with relevant time information. For instance, if the input comprises a specific sample of a biomaterial, the experiment may produce a differently-numbered sample of the same organism. In addition, the experiment output 920 may comprise results in the form of various data entities (such as the data sets shown in Figures 6A and 6B, or documents or spreadsheet files). The experiment output 920 may also comprise a phenotype classification and/ or a genotype classification in data entities.

Data traceability will be improved by the fact that the experiment input 914 and experiment output 920 have a relevant time, as denoted by items 915 and 921 respectively. The times 915, 921 indicate times when the relevant biochemical event, such as sample taking, perturbation, or the like, took place. Data traceability will be further described in connection with Figures 11A and 11B.

An experiment has also a target 930, which is typically a biomaterial 932 (eg population, individual, reagent or sample) but the target of in-silico experiments may be a data entity 934.

The method entity 910 has a relation to a method description 912 that describes the method. The loop next to the method description 912 means that a method description may refer to other method descriptions.

The experiment input 914 and experiment output 920 are either specific biomaterials 916, 922 or data entities 918, 924, which are the same data elements as the corresponding elements in Figure 2. If the experiment is a wet-lab experiment, the input and output biomaterials 916, 922 are two instances (same or different) of biomaterial 210 in Figure 2. For example, they may be two specific samples 210-4.

Because the biochemical information (reference numeral 200 in Figure 2) and the project information are described with common data entities, the project manager is able to track the history of each piece of information. It is also able to monitor productivity as an amount of added information per resource (such as person year).

The experiment project manager preferably comprises a project editor having a user interface that supports project management functionality for project activities. That gives all the benefits of standard project management that are useful in systems biochemical projects as well.

A preferred implementation of the project editor is able to trace all biomaterials, their samples and all the data through the various experiments including wet-lab operations and in-silico data processing.

An experiment project can be represented as a network of experiment activities, target biomaterials and input or output deliverables that are biomaterials or data entities.

In terms of complexity, Figure 9A shows a worst-case scenario. Few, if any, real-life experiments comprise all the elements shown in Figure 9A. For instance, if the experiment is a medical or biochemical treatment, the input and output sections 914, 920, typically indicate a certain patient or a biochemical sample. An optional condition element may describe the condition of the patient or sample before treatment. The output section is a treated patient or sample.

In case of sampling the input section indicates a biomaterial to be sampled, and the output section indicates a specific sample. In case of sample manipulation the input section indicates a sample to be manipulated and the output section indicates the manipulated sample. In a combination experiment the input section indicates several samples to be combined and the output section indicates the combined, identified sample. Conversely, in a separation experiment the input section indicates a sample to be separated and the output section indicates several separated, identified samples. In a measurement experiment the input section indicates a sample to be measured and the output section is a data entity containing the measurement results. In a classification experiment the input section indicates a sample to be classified and the output section indicates a phenotype and/or genotype. In a cultivation experiment the input and output sections indicate a specific population, and the equipment section may comprise identities of the cultivation vessels.

In order to describe complex experiments, there may be experiment binders (not shown separately) that combine several experiments in a manner which is somewhat analogous to the way the pathway connections 700, 720, 730 combine various pathways.

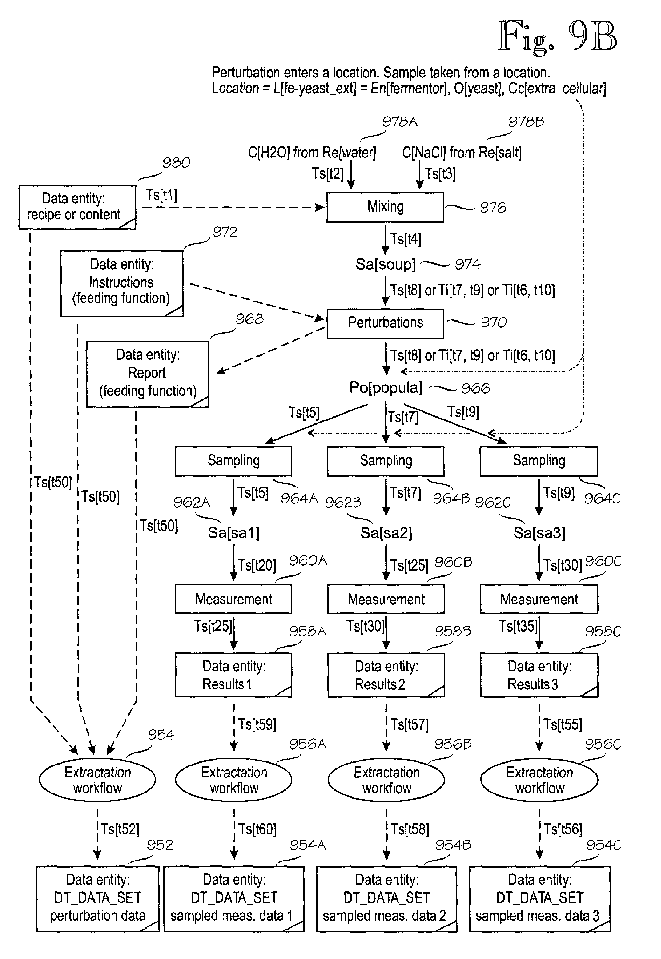

Figure 9B illustrates creation of a project plan from a set of desired results. The project plan shown in Figure 9B is a representative sample of project plans that can be created with the system shown in Figure 9A. As shown in Figure 9A, an experiment input 914 is processed by a method 910 to an experiment output 920, which may be applied as experiment input to another method, and so on. In Figure 9B, rectangles like mixing 976 and perturbations 970 represent methods, while biomaterials, such as sample 974 and population 966, represent experiment input and/or output.

If the project plan shown in Figure 9B is created on a graphical user interface by a designer, it is self-explanatory. But what makes it interesting is that the systematic project structure shown in Figure 9A makes it possible to provide the IMS with a routine for automatically creating a project plan, or at least some of its intermediate acts, from a set of desired results.

Assume that a researcher wishes to obtain four data sets, namely perturbation data 952 that describes a set or perturbations to be entered into a population 966 and sampled measurement data 954A - 954C from the population 966. The population 966, labelled Po[popula] and specified in the data sets 952 and 954A - 954C, is an instance of a biomaterial experiment target 932 and 930 (see Figure 9A). It will be affected by perturbations 970 at times specified in data set 952. The perturbation 970 is prepared by a mixing experiment 976 derived from perturbation variable data of the data set 952 and a method description 912 of the mixing method 910, with a recipe data entity 980 as experiment input 918 and biomaterials 978A and 978B as experiment input 916 and a sample 974 as a biomaterial experiment output 922. Three sampling operations 964A - 964C will create three samples 962A - 962C of the experiment target 966, ie Po[popula], at times specified in the data sets 954A - 954C. The samples 962A - 962C are analyzed in measurement experiments 960A - 960C derived from measurement variable data of data sets 954A - 954C and method descriptions 912 of the measurement methods 910. The samples 962A - 962C are instances of experiment inputs 916 (see Figure 9A) and the data entities 958A - 958C are instances of experiment outputs 924.

In this way, experiment targets 930 and intermediate experiments 904 and their inputs 914 and outputs 920 with required timing 915 and 921 can be determined by the information of data sets 952 and 954A - 954C and predefined methods 910 and method descriptions 912 when variable data of data sets are mapped into methods in method descriptions 912.

The problem faced by the logic for creating automatic project plans is how to determine the intermediate steps from data sets 954A - 954C to the population 966. The logic is based on the idea that in a typical research facility, any type of measurement data can only be created by a limited set of measurement methods. Assume that the first data set 954A contains data for which there is only one method description 912 (see Figure 9A). In such a case that method, ie measurement 960A, can be selected automatically. If the remaining data sets 954B and 954C contain types of data that can be obtained by several measurement methods, the logic can offer the potential method candidates for selection by the user. But as soon as the user has selected appropriate measurement methods 960B and 960C, the logic can infer that three samples 960A to 960C are needed for the three measurements. Since three samples are needed, three sampling operations 964A to 964C of the population 966 are needed as well, since sampling is the only operation that produces a sample. The same idea can be applied to derive specific mixing or other preparation experiments for perturbation experiments targeted for the research target. Thus the systematic object-based project description shown in Figure 9A can be used by a logic for automatically creating at least some intermediate acts in a project plan as shown in Figure 9B.

Furthermore, the logic can also infer advantageous time stamps for the acts of the project plan. As shown in Figure 9B, each act has an associated time stamp Ts[time]. Assume that the researches wishes to determine beforehand an optimized set of time stamps for the sampling of population 966. The time stamps are shown as Ts[t5], Ts[t7] and Ts[t9]. The logic can use the kinetic laws described in connection with the pathways (Figures 7A to 8) and carry out a simulation of what will happen in the population 966 in response to the perturbations 970. Most likely the simulation will result in an activity that takes some time to start, then peaks and finally levels off. The researcher or the logic itself can determine an optimized set of time stamps such that all the major phases (start, peak, level-off) of the activity will be adequately covered by measurements.

**Biomaterial descriptions**

Figure 10 shows an example of an object-based implementation of the biomaterials section of the IMS. Note that this is but one example, and many biomaterials can be adequately described without all elements shown in Figure 10. The biomaterial section 210, along with its sub-elements 210-1 to 210-4, and the location section 214 with its sub-elements 214-1 to 214-5 have been briefly described in connection with Figure 2. In addition to the previously-described elements, Figure 10 shows that a biomaterial 210 may have a many-to-many relation to a condition element 1002, a phenotype element 1004 and to a data entity element 1006. An optional organism binder 1008 can be used to combine (mix) different organism. For example, the organism binder 1008 may indicate that a certain population comprises x per cent of organism 1 and y per cent of organism 2.

A loop 1010 under the organism element 214-1 means that the organism is preferably described in a taxonomical description. The bottom half of Figure 10 shows two examples of such taxonomical descriptions. Example 1010A is a taxonomical description of a specific sample of coli bacteria. Example 1010B is a taxonomical description of white clover.

The variable description language described in connection with Figures 3A to 3C can be used to describe variables relating to such biomaterials and/or their locations. Example:

V[concentration]P[P53]U[mol/l]Id[Patient X]L[human cytoplasm]=0.01.

A benefit of this kind of location information is an improved and systematic way to compare locations of samples and locations of theoretical constructs like pathways that need to be verified by relevant measurement results.

Another advantage gained by storing the biomaterials section substantially as shown in Figure 10 relates to visualization of data. For example, biomaterials can be replaced with their phenotypes. An example of such replacement is that certain individuals are classified as "allergic", which is far more intuitive to humans than a mere identification.

**Data traceability**

Data traceability is based on the time information 915 and 921 associated with experiment inputs and outputs 914 and 921, respectively (see Figure 9A). Figures 11A and 11B demonstrate data traceability in the light of two examples. Figure 11A shows a sampling scenario. All samples are obtained from a certain individual A, denoted by reference number 1102. Reference number 1104 generally denotes four arrows each of which corresponds to a certain sampling at a certain time. For example, at time 5 a sample 4 is obtained, as indicated by reference numeral 1106. Using the VDL shown in connection with Figures 3A to 4, sample 4 at time 5 can be expressed as Sa[4]T[5]. The expression Sa[4]T[5] = Id[A]T[5] means that sample 4 was obtained from individual A at time 5.

At time 12 two further samples are obtained from sample 4. As shown by arrow 1108, sample 25 is obtained from sample 4 by separating the nuclei. Reference numeral 1112 denotes an observation (measurement) of sample 25, namely the concentration of protein P53, which in this example is shown as 4.95.

Figure 11 B illustrates data traceability in a scenario in which a perturbation is caused by administering certain compounds to an individual B, 1150. As shown by reference numerals 1152 to 1158, a 10-gram dose of compound abcd is applied to sample 40 at time 1, and that sample is administered to individual B at time 6. Reference numeral 1160 denotes administration of mannose to individual B at time 5. The bottom half of Figure 11 B is analogous to Figure 11A, and a separate description is omitted.

Showing images such as those contained in Figures 11A and 11 B helps users to understand what the observations are based on. Benefits of improved data traceability include better understanding of relevant timing of experiments inputs and outputs as well as reduction of errors and easier explanation of anomalies.

It should be understood that real-life cases can be far more complex than what can reasonably be shown on one drawing page. Thus Figures 11A and 11B show the principle of data traceability. In order to support complex cases, the visualization logic should be preceded by user-activated filters that let users see only the topics of interest. For example, if a user is only interested in sample 25 shown in Figure 11A, only the chain of events (samples) 1102 - 1106 - 1110 - 1112 can be displayed.

**Workflow descriptions**

Figure 12A shows an information-entity relationship for describing and managing workflows of virtually arbitrary complexity within the IMS. A workflow 1202 may contain other workflows, as indicated by arrow 1203. The lowest level workflow contains a tool definition 1208. Each workflow has an owner user 1220. Each workflow belongs to a project 1218. (Projects were discussed in connection with Figures 9A and 9B.)

Tools are defined in terms of tool name, category, description, source, pre-tag, executable, inputs, outputs and service object class (if not the default). This information is stored in a tool table or database 1208.

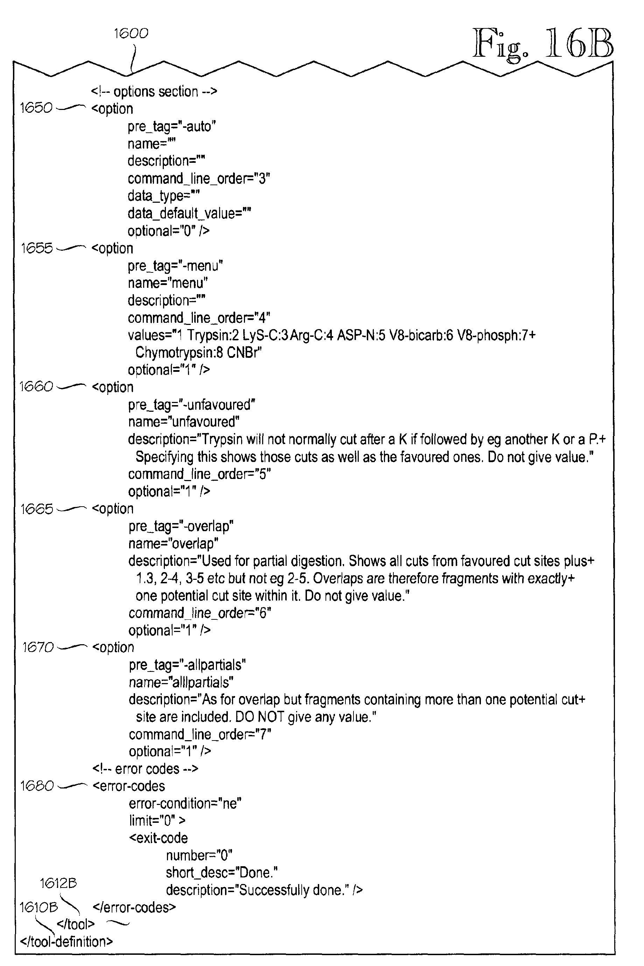

An input definition includes pre-tag, id number, name, description, data entity type, post-tag, command line order, optional-status (mandatory or optional). This information is stored into the tool input binder 1210 or tool output binder 1212. In a real-life implementation, it is convenient to store the tool 1208, the tool input binder 1210 and tool output binder 1212 in a single disk file, an example of which is shown in Figures 16A and 16B.

The data entity types are defined to the system in terms of data entity type name, description, data category (eg file, directory with subdirectories and files, data set, database, etc). There are several data entity types that belong to the same category but having different syntax or semantics and consequently belong to different data entity type for compatibility rules of existing tools. This information is stored in data entity type 1214. Tool server binder 1224 indicates a tool server 1222 in which the tool can be executed. If there is only one tool server 1222, the tool server binder 1224 can be omitted.

Typed data entities are used to control the compatibility of different tools that might be or might not be compatible. This gives the possibility to develop a user interface in which the systems assists users to create meaningful workflows without prior knowledge about the details of each tool.

The data entity instances containing user data are stored in data entity 1216. When workflows are built the relevant data entities are connected to relevant tool inputs through workflow inputs 1204 or workflow outputs 1206. Reference numeral 1200 generally denotes the various data entities, which in real-life situations constitute actual instances of input or output data.

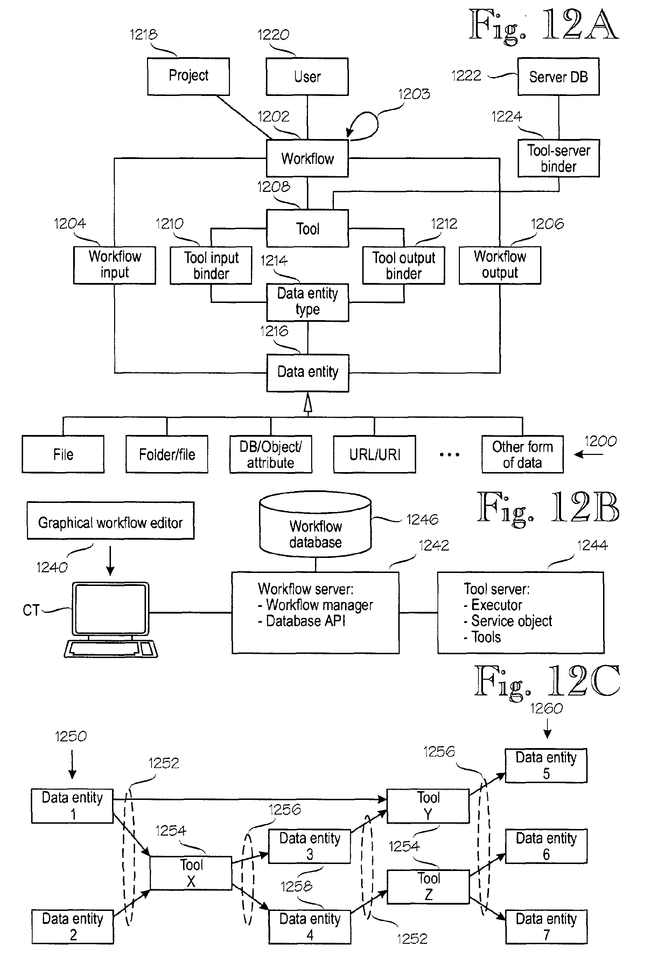

Figure 12B shows a client-server architecture comprising a graphical workflow editor 1240 being executed in a client terminal CT. The graphical workflow editor 1240 connects via a workflow server 1242 to an executor and a service object in a tool server 1244. The graphical workflow editor 1240 is used to prepare, execute and monitor and view workflows and data entities communicating with a workflow database 1246. The workflow server 1242 takes care of executing workflows by using one or more tool servers 1244. The address of the relevant tool server can be found from the server table 1222 (Figure 12A).

Each tool server 1244 comprises an executor and a service object that is able to call any standalone tool installed on the tool server. The executor manages executing all the relevant tools of a workflow with relevant data entities through a standardized service object. The service object provides a common interface for the executor to run any standalone software tool. Tool-specific information can be described in an XML file that is used to initialize metadata for each tool in the tool database (item 1208 in Figure 12A). The service object receives the input and output data and by using the tool definition information, it can prepare the required command line for executing the tool.

A workflow/tool manager as shown in Figures 12A and 12B easily integrates legacy tools and third-party tools. Other benefits of the workflow/tool manager include complete documentation of workflows, easy reusability and automatic execution. For instance, the workflow/tool manager can hide the proprietary interfaces of third-party tools and substitute them with the common GUI of the IMS. Thus users can use the functions of a common graphical user interface to prepare, execute, monitor and view workflows and their data entities.

Note that Figure 12A shows an information-entity relationship that shows the mutual relations between different types of entities, tools etc. Figure 12A shows, for example, that a tool input binder 1210 defines a relation between an input of a tool 1208 and a data entity type 1214, which may or may not be the same type as the one that represents the tool's output as defined by the tool's output binder 1212.

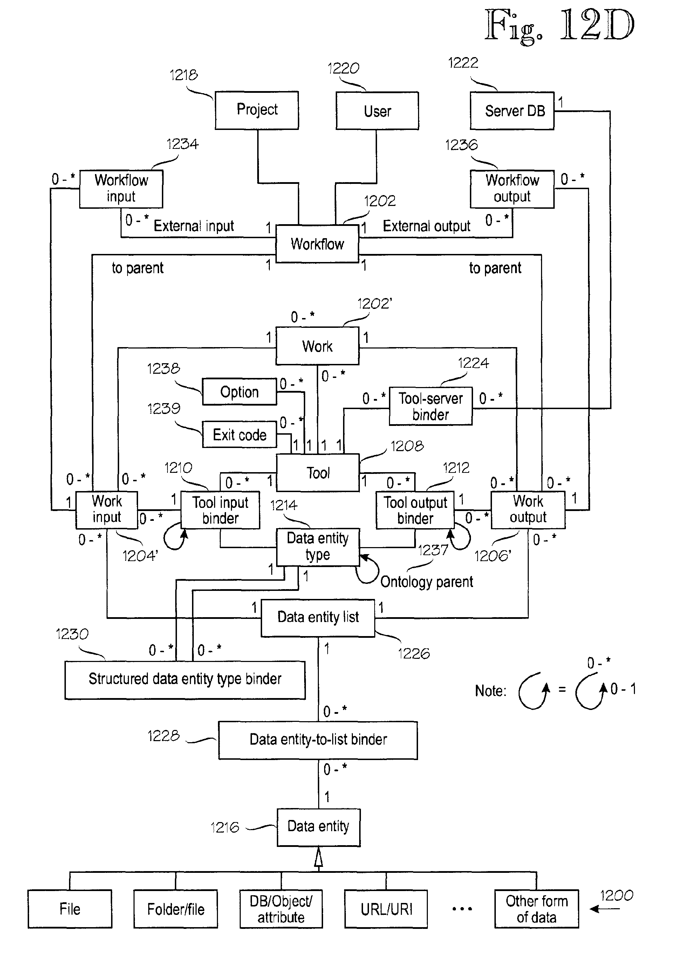

Figure 12C shows the interrelation of tools and data entities from an end user's point of view. The available tools and data entities can be combined as logical networks (workflows) of arbitrary complexity, wherein one tool's output is connected to the next tool's input, and so on. Note that each tool needs to be defined only once. For each instantiated execution of a tool, there is a child workflow 1202 (or work 1202' in Figure 12D) that can be created for each graphical "tool" icon. Reference numeral 1250 denotes input data entities, which in this example are data entities 1 and 2. Reference numerals 1252 denote workflow inputs. Reference numerals 1254 denote the tools X, Y and Z used in this workflow. In this example the workflow inputs 1252 bind data entities 1 and 2 to child workflows using tool X and Y, and data entities 1, 3 and 4 also to child workflows using tool Y and Z. Reference numerals 1256 denote workflow outputs, which in this example bind data entities 3 and 4 to child workflows using tool X and data entities 5, 6 and 7 to child workflows using tools Y and Z. Reference numerals 1258 denote intermediate data entities that constitute the output from a child workflow that calls tool X, providing inputs to another child workflow that calls tools Y and Z. Reference numeral 1260 denotes output data entities, which in this example are data entities 5, 6 and 7. Each workflow input 1252 or workflow output 1256 is an instance of the respective class 1204, 1206 shown in Figure 12A. Tool input binders 1210 and output binders 1212 are used in a graphical user interface to assist users in building workflows, by connecting tools and data entities with correct data entity types for each input or output.

As shown in Figure 12C, the workflow inputs 1252 or workflow outputs 1256 collectively define a data flow network from the input data entities 1250 to its output data entities 1260, such that each workflow input 1252 connects a specific data entity to an input of a tool 1254 and each workflow output 1256 connects the tool's output to a specific data entity, which may be an intermediate data entity 1258 or an output data entity 1260. The tools are executed on the basis of topological sorting of workflows. Such workflows are most useful for complex tasks that need to be repeated over and over again with different inputs.

The embodiment shown in Figure 12C hides certain abstract concepts, such as child workflows, workflow inputs and outputs but shows more concrete things, such as data entities, tools, tool inputs and tool outputs.

Figure 12D shows an enhanced version of the information-entity relationship shown in Figure 12A. Items with reference numerals lower than 1224 were described in connection with Figure 12A and will not be described again. The embodiment shown in Figure 12D has several enhancements over the one shown in Figure 12A.

One enhancement consists of the fact that the hierarchical workflow 1202, 1203 of Figure 12A has been divided into a workflow 1202 and work 1202', wherein the work 1202' is at the bottom level of the hierarchy and does not contain any child workflows. A workflow's external input and output are the workflow defined by workflow input 1236 and workflow output 1238, respectively. The external input and output of the workflow define the overall input and output, without any internal data entities that are used only within the workflow. The workflow's internal data entities are defined by work input 1204' and work output 1206'.

Another enhancement consists of the fact that the work input 1204' and work output 1206' are not connected to a data entity 1216 directly but via a data entity list 1226 which, in turn, is connected to the data entity 1216 via a data entity-to-list binder 1228. A benefit of this enhancement is that a work's input or output can comprise lists of data entities. This simplifies end-user actions when multiple data entities are to be processed similarly. Technically speaking, the data entity list 1226 specifies several data entities as an input 1204' or output 1206' of a work, such that each data entity in the list is processed by a tool 1208 separately but in a coordinated manner.

A third enhancement is a structured-data-entity-type binder 1230 for processing structured data entities, such as the data sets 610 and 620 shown in Figures 6A and 6B. Such data sets consist of four data entities (describing common, rows, columns and value matrix) each, and the structured data entities can be defined by the structured-data-entity-type binder 1230. Thus the end-users are not concerned with interrelations of the data entities.

Moreover, each tool 1208 may have associated options 1238 and/or exit codes 1239. The options 1238 may be used to enter various parameters to the software tools, as is well known in connection with script file processing. The options 1238 will be further discussed in connection with Figures 16B and 16B (see items 1650 - 1670 and 1696 - 1697). The exit codes (or error codes) 1239 can be used to convey the termination status of a tool back to a user via the service object, the executor, the workflow server and the graphical workflow editor. For instance, if the operation of a tool is interrupted because of some kind of processing error, there is little point in a subsequent tool to carry out its intended task but let the user know the termination status. Examples of exit codes will be shown in Figure 16B (see section 1680).

Yet another optional enhancement shown in Figure 12D is that the type definition 1214 contains an ontology definition. A benefit of the ontology definition is that the type checking of a tool to/from a data entity does not have to succeed literally but conceptually. For example, a tool's definition may specify that the tool outputs files in "Rich Text Format", while another tool's definition specifies that the tool processes (inputs) "text" files. A literal comparison of "text" and "Rich Text Format" will fail but an appropriately configured ontology definition is able to indicate that "Rich Text Format" is a subclass of "text" files, whereby the ontological type checking succeeds.

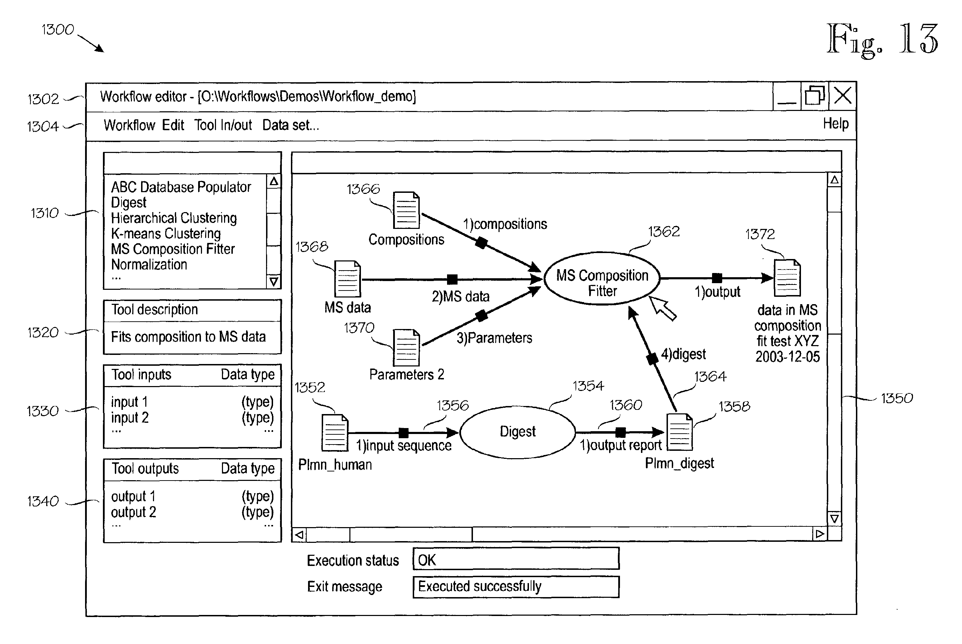

Figure 13 shows an exemplary user interface 1300 for a workflow manager. A title bar 1302 and menu bar 1304 are self-evident to persons familiar with graphical user interfaces. A tool selector box 1310 lists all available tools. A tool descriptor box 1320 shows a description for the selected tool. A tool input box 1330 and tool output box 1340 list and describe, respectively, the selected tool's inputs and outputs. A graphical workflow editor box 1350 shows the contents of the workflow being edited, ie the interrelation of the various data entities and tools, in a graphical form. The graphical workflow editor box 1350 shows, in principle, similar subject matter as was shown in Figure 12C, but in Figure 12C the emphasis was on logical relations between tools, data entities and binders, while Figure 13 shows a more realistic view of an actual user interface. In this example, data entity 1352 is an input of tool 1354, as shown by the connector arrow 1356. The output of tool 1354 is data entity 1358, as shown by connector arrow 1360. Data entity 1358, which is the output of tool 1354 will be used as one of the inputs of tool 1362, as shown by connector arrow 1364. Tool 1362 has three other inputs 1366, 1368 and 1370. In this example, inputs 1366 and 1368 are data entities, and input 1370 contains various optional or user-settable parameters. Another way of entering parameters, particularly non-optional parameters, will be shown in Figure 16B (see option section 1650 - 1670 in configuration file 1600). The output of tool 1362 is data entity 1372, which is also the output of the entire workflow. Actually, the workflow being edited in the workflow editor box 1350 may be a child workflow of some parent or upper-level workflow, as shown by arrow 1203 in Figure 12A, and the output of that child workflow will be used as an input in that upper-level workflow.

The elements in Figures 13 relate to those in Figure 12A or 12D as follows. Each data entity 1352, 1358, shown with a "file" type icon, such as icon 1352, is an instance of the data entity class 1216 in Figure 12A or 12D. Tools shown in the tool selector box 1310 are instances of the tool class 1208 in Figure 12A or 12D. They can be selected from the tool selector box 1310 when instantiating their potential executions as child workflows in Figure 12A or works in Figure 12D. Child workflows or works of relevant tools 1354 and 1362 are used in the workflow being edited as instances of child workflows 1202 in Figure 12A or as instances of works 1202' in Figure 12D.

The parent workflow being edited is an instance of workflow class 1202. The arrows 1356, 1364, etc., created by the graphical user interface in response to user input, represent instances of a work or workflow input 1204', 1204. These arrows connect a data entity as an input to a work that will be done by executing the tool when the workflow is executed. The relevant tool is indicated with a "tool" type icon, such as icon 1354. The tool input binders 1210 enable type checking of each connected instance of a data entity. The arrows 1360 represent instances of a work or workflow output 1206, 1206'. These arrows connect a data entity as an output from a work that will be done by executing the tool when the workflow is executed. The relevant tool is indicated with a "tool" type icon. The tool output binders 1212 enable type checking of each connected instance of a data entity.

A benefit of this implementation is that the well-defined type definition shown in Figures 12A and 12D supports thorough type-checking which ensures data reliability and integrity. In the user interface 1300, the type checking may be implemented such that an interactive connection between a data entity and a tool can only be performed if the type check is successful. In addition, the data entity types may be shown in the selected tool's input box 1330 and output box 1340.

Again, abstract concepts, such as child workflow and workflow input, workflow output, work input and work output are hidden from the users of the graphical user interface, but more concrete elements, such as data entities, tools, tool inputs and tool outputs are visualized to users as intuitive icons and arrows.

In case of quantitative data, the data entities 1216, 1352, etc. are preferably organized as data sets 610, 620, and more particularly as variable value matrixes 614, 624, that were described in connection with Figures 6A and 6B. A benefit of the variable value matrixes 614, 624 in this environment is that the software tools, which may be obtained from several sources, only have to process arrays but no dimensions or matrix row or column descriptors.

The graphical user interface preferably employs a technique known as "drag and drop", but in a novel way. In conventional graphical user interfaces, the drag and drop technique works such that if a user drags an icon of a disk file on top of a software tool's icon, the operating system interprets this user input as an instruction to open the specified disk file with the specified software tool. But the present invention preferably uses the drag and drop technique such that the specified disk file (or any other data entity) is not immediately processed by the specified tool. Instead, the interconnection of a data entity to a software tool is saved in the workflow being created or updated. Use of the familiar drag and drop metaphor to create saved workflows (instead of triggering ad-hoc actions) provides several benefits. For example, the saved workflows can be easily repeated, with or without modifications, instead of recreating each workflow entirely. Another benefit is that the saved workflows support tracing of workflows.

Dedicated tool input and output binders make it possible to use virtually any third-party data processing tools. The integration of new, legacy or third-party tools is made easy and systematic.

The systematic concept of workflows hides the proprietary interfaces of third-party tools and substitute the proprietary interfaces with a common graphical user interface of the IMS. Thus users can use the functions of a common graphical user interface to prepare, execute, monitor and view workflows and their data entities. In addition, such a systematic workflow concept supports systematic and complete documentation, easy reusability and automatic execution.

The concept of data entity provides a general possibility to experiment with any data. However, the concept of data entity type makes possible to understand, identify and control the compatibility of different tools. Organization of quantitative data as data sets, each of which comprises a dimensionless variable value matrix, provides maximal compatibility between the data sets and software tools from third parties, because the tools do not have to separate data from dimensions or data descriptors.

Because of the graphical interface, researchers with a biochemical expertise can easily connect the biologically relevant data entities to or from available inputs or outputs and get immediate visual feedback. Inexperienced users can reuse existing workflows to repeat standard workflows merely by changing the input data entities. The requirement to learn the of the syntactic and semantic details of each specific tool's command line can be delegated to technically-qualified persons who integrate new tools to the system. This benefit stems from the separation of the tool definitions from the workflow creation. Biochemical experts can concentrate on workflow creation (defined in terms of data entities, works, workflows, work inputs, workflow inputs, work outputs, workflow outputs), while the tool definitions (tools, tool input binders, tool output binders, options, exit codes), are delegated to Information-technology experts.

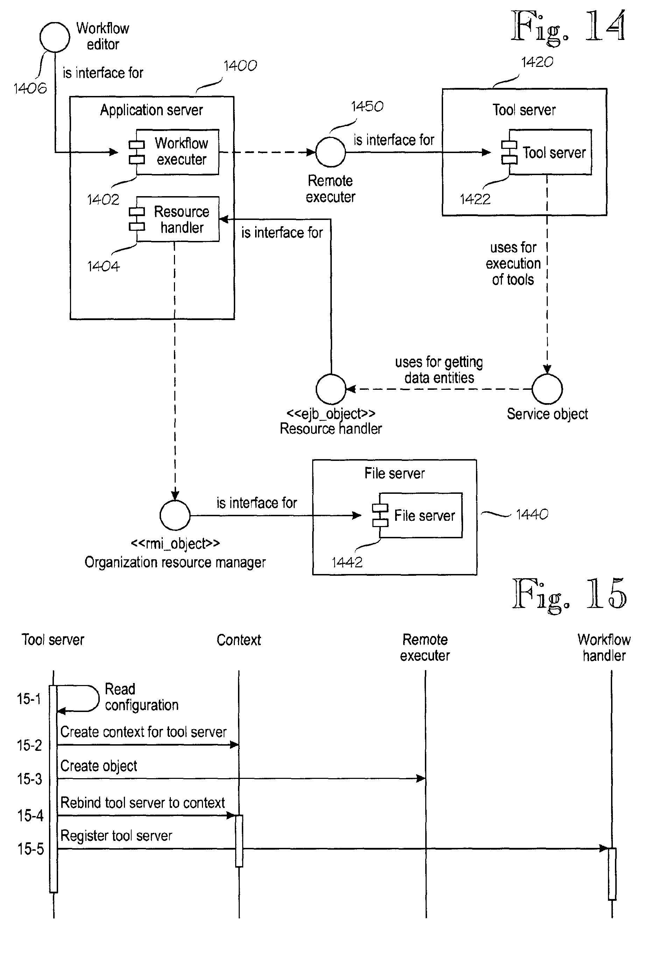

Figures 14 to 19 relate to implementation details of a preferred workflow execution environment. As was briefly stated in connection with Figure 12A, and shown by the arrow 1203 that begins and ends at workflow 1202, the workflows may be hierarchical. This means that when a user creates a new workflow in the workflow manager, a root workflow is created. The user can then create child workflows ("work 1202' in Figure 12D) that are connected to the root workflow. In the following, the word 'root' may be omitted. Each child workflow is associated to one tool only. That tool defines the allowed inputs and outputs of the workflow and the tool is used for executing the child workflow. When the user creates a child workflow, the required inputs and outputs are also created based on the definitions in the tool's interface. The user may add optional inputs and outputs if the tool interface supports additional inputs and/or outputs. After adding the child workflows, the user can graphically connect the output of one child workflow to the input of another child workflow, provided that the input and output types match. This means that the user at the graphical user interface creates a stored association in which the output of a tool (=child workflow) becomes the input of a next tool. Each root workflow is that a collection of such output-to-input associations. Although the root workflows (collections of stored associations) can be arbitrarily complex, their use is extremely simple, since no intervening manual operations are needed.

Figure 14 shows an object-based implementation of the workflow execution environment. This implementation comprises three servers, namely an application server 1400, a tool server 1420 and a file server 1440. The three servers are to be understood as three logical servers but not necessarily three server computers. The system preferably comprises only one logical application server 1400 but it may comprise several tool servers 1420. In order to eliminate ambiguity, each tool server can register itself to only one application server. A user can execute an entire workflow or only a child workflow.

Each tool in the tool server 1420 essentially consists of two parts: an executable part and an interface definition. The executable (part) is a collection of program instructions for executing a certain data-processing action. The interface definition is preferably implemented as an XML file (XML = extendible mark-up language).

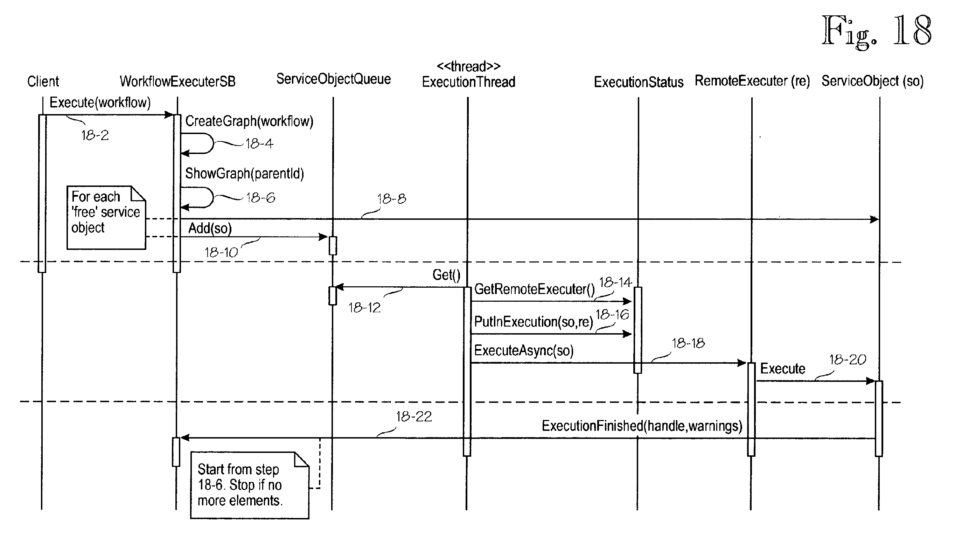

The application server 1400 comprises a workflow executer component 1402 that actually executes workflows. The workflow executer component 1402 is a component-based implementation of the workflow server 1232 shown in Figure 12B. A workflow editor 1406 or some other client calls the execute method in the workflow executer interface. This call is an asynchronous call. Depending on whether the client wishes to execute the entire workflow or only a child workflow, an identifier of the root workflow or the child workflow, respectively, is given as a parameter of the call. Details of this process will be described in connection with Figure 18.

For each call from a client, the application server 1400 first creates a workflow graph from the child workflows. If the workflow is a child workflow, the graph consists of that workflow. Otherwise the graph contains zero or more child workflows. After that, the application server scans through graph and picks up any child workflows whose inputs are not outputs of any other workflow. Such child workflows are added to a workflow queue. A workflow executer 1402 distributes the execution of the child workflows in the queue to the tool server(s) 1420 beginning from the first child workflow at the beginning of the queue. The tool server executes the child workflow asynchronously and reports the completion of the execution. When the execution is completed, the child workflow is removed from the workflow graph, after which the graph is rescanned and the above procedure is repeated as long as the graph contains any child workflows.

Whenever a tool server 1420 is started, it registers itself in the application server 1400. The application server maintains information on each tool server in a database. Accordingly, the application server can connect to an available tool server via a server name table. Before a tool can be executed in the tool server the tool must be installed in an appropriate tool server.

The installation process involves reading tool definition information from a tool definition file, an example of which will be shown in Figure 16, and updating relevant tool related database tables in Figure 12 A or 12D based on the tool definition information. When the tool is installed, the executables are first copied to their appropriate locations, after which the tools is registered in the application server 1400. Registering per se is known in the art, and it means that information on the tool, including information on its interfaces, is recorded in the database unless such information already exists in the database. The information in the database may indicate the tool server the tool resides in and miscellaneous installation details (who, when, where, etc.) Figure 15 shows an exemplary procedure for starting a tool server. In step 15-1, the tool server 1420 reads its configuration information from a tool server configuration file. The properties of the parameters read from the tool server configuration file are saved as global variables, whereby they are available to all processes. In step 15-2, the tool server creates a separate context for tool servers. For example, the context may be of the form "com/company/bms/toolserver". In step 15-3, the tool server creates a RemoteExecuter object, shown as item 1450 in Figure 14. In step 15-4, the a binding of the tool server and the newly-created context is made. Finally, in step 15-5, the tool server registers itself to a workflow executer 1402.

Figures 16A and 16B, which form a single logical drawing, show an exemplary tool definition file 1600. The tool definition file is used to populate the tool related information described in Figures 12A and 12D into the database when the tool is installed into the tool server. Later this information is taken from the database when actual workflows are created or executed or monitored. The content of the tool definition file and the purpose of its content is explained as follows.

Section 1605 is a comment section that is ignored by computers. Lines 1610A and 1610B delineate a tool definition, which constitutes the body of the tool definition file 1600. Lines 1612A and 1612B delineate a definition for a single tool. Section 1615 contains overall parameters of the tool, named "Digest" in this example. For example, section 1615 indicates that the tool has two inputs and one output. Lines 1620 and 1630 begin, respectively, definitions of the first and second input. Line 1640 begins a definition of the tool's output. Lines 1650, 1655, 1660, 1665 and 1670 begin definitions for five option sections. Line 1680 begins an error/exit code definition.

Figure 16C shows a script 1690 generated automatically, under control of the IMS, and populated from the tool definition file 1600. The first element 1691 is the relevant tool's name, as obtained from section 1615 of the tool definition file 1600.

The second element 1692 is an input pre-tag, which in case of this particular tool precedes the name 1693 of the input data entity. The pre-tag is obtained from section 1620 of the tool definition file 1600. The place of each input with its pre-tag or post-tag in the command line is defined by the command-line-order field of each input. This order number is relative indicating the place of each element if all the optional command line elements (inputs, outputs, options) are present. There might be an input data entity visible in the graphical user interface containing option data for several options that are not given in one place of the command line. In this case the command-line-order is not relevant for this input. This is the case of section 1630.

In an analogous manner, the output data entity 1695 is preceded by an output pre-tag, as determined by section 1640 of the tool definition file. The command-line-order of each output indicates the place of the output data entity with its pre-tags and post-tags.

The potential options of each tool are described with their command-line-order fields. Two parameters 1696 and 1697, obtained from sections 1650 and 1660 of the configuration file, respectively, control the action of the tool. From the point of view of the users, all relevant options are in one input data entity and the IMS places them automatically to the correct places in the command line.

In prior art IMS systems, scripts such as the script 1690 shown here, are generated manually with text-editing tools, which requires programming skills or at least considerable experience with each software tool.

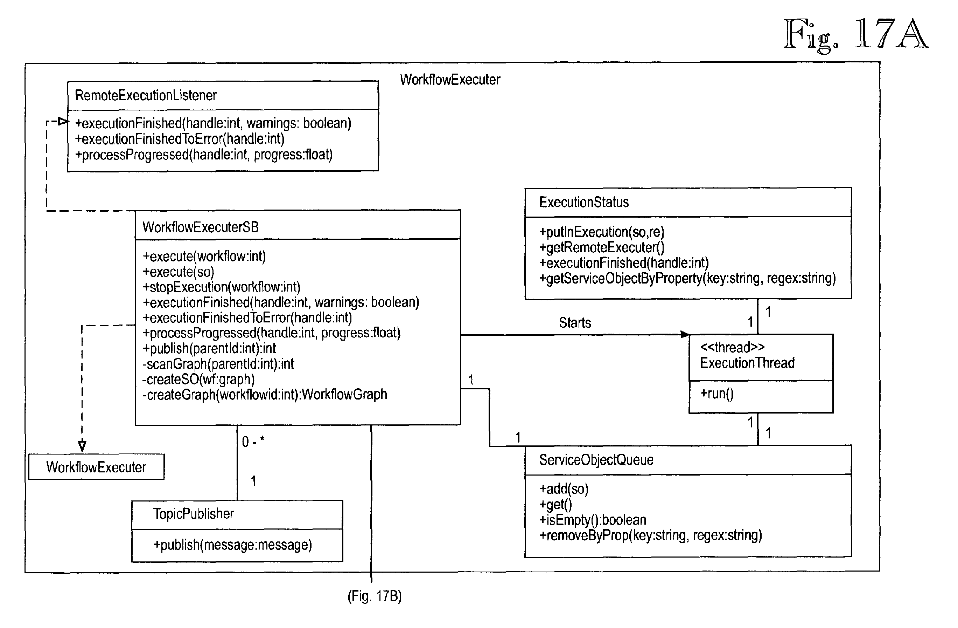

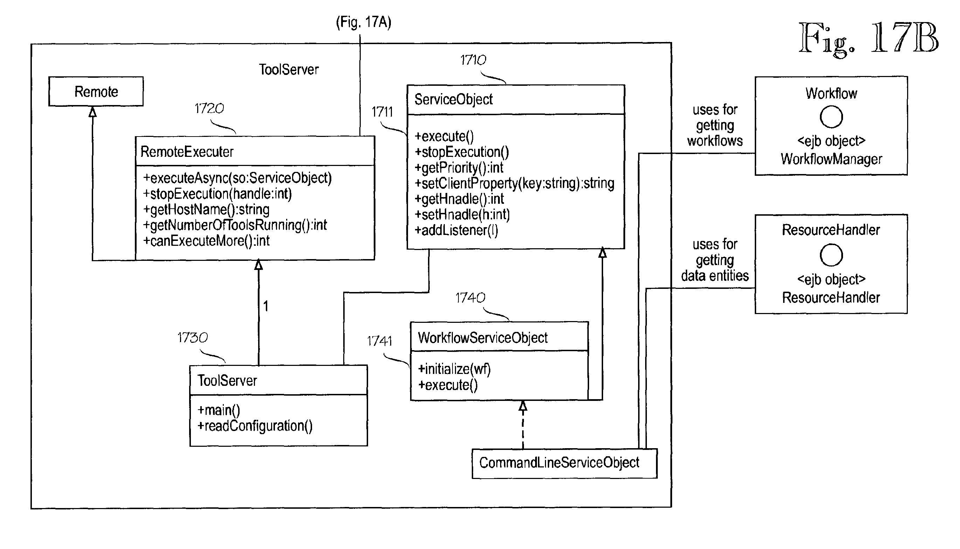

Figures 17A and 17B, which form a single logical drawing, show a class diagram for an exemplary tool execution environment. Each service object 1710 is a general interface that executes a meaningful service, such as some amount of calculation, by calling a specific tool with an appropriate dynamically created command line for a specific work. A remote executer 1720 is a remote interface for executing the service objects 1710. The tool server, shown here as 1730, is called by the workflow executor 1402 in the application server (item 1400 in Figure 14) through the remote executor 1450. The service may be implemented as a remote method invocation (RMI) service for example. The service object interface 1711 preferably comprises the following methods:

- *execute()* executes the service object synchronously; when the execution is finished the service object is responsible for reporting the status of the execution through a status listener (RemoteExecutionStatusListener).
- *stopExecution()* stops the execution as fast as possible, in which case the service object must not inform the status listener.
- *getPriority():int* returns the priority of the execution.
- *setPriority(p:int)* sets the priority of the execution.
- *setHandle(h:int)* sets a handle, which acts as an identifier of the service objects; each service object creator must create a handle, such as a unique number in the database.
- *getHandle():int* returns the handle.
- *setListener(I:RemoteExecutionListener)* sets a listener for the service.

Each workflow service object 1740 comprises a workflow service object interface 1741, which preferably comprises the following methods:

- *initialize(wf:workflow)* saves an identifier of the workflow to an instance variable.
- *execute()* retrieves the workflow and tool interface from the database, checks the syntax of the workflow, retrieves the relevant input from the file server, creates a command line and applies it to the tool, thus causing its execution.

Based on the two exemplary interface definitions 1711 and 1741, the skilled reader can implement the remaining interfaces shown in Figures 17A and 17B.

Figure 18 shows exemplary process steps to execute a workflow. In step 18-2, a call from the client invokes the workflow executer $$. As shown by loop 18-4, the workflow executer creates a workflow graph from the child workflows. In this context, the 'graph' should be understood as a computerized structure rather than a visual representation. If the workflow is a child workflow, the graph consists of that workflow. Otherwise the graph contains zero or more child workflows. In step 18-6 the application server scans through graph and picks up any child workflows whose inputs are not outputs of any other workflow. Then, as shown by steps 18-8 and 18-10, the workflow executer $$ creates service objects from such workflows and adds them to a queue of service objects ("workflow queue"). In step 18-12, the execution thread gets the next service object from this queue. In step 18-14, the execution thread gets an optimal remote executer from execution status $$. In step 18-16, the execution thread updates the execution status with information on the service object and remote executer. In step 18-18, the execution thread invokes the remote executer, which executes the service object in step 18-20. In step 18-22, when the execution of the service object is completed, the service object notifies the remote execution listener. If the completion was successful, the newly-executed workflow is removed from the queue and workflow graph, then the graph is rescanned and the above procedure is repeated, beginning at step 18-4. If the execution encounters an error, the workflow execution is stopped.

The invention and its preferred embodiments provide numerous advantages. Dedicated tool input and output binders make it possible to use virtually any third-party data processing tools. The integration of new, legacy or third-party tools is made easy and systematic.

The systematic concept of workflows according to the invention hide the proprietary interfaces of third-party tools and substitute the proprietary interfaces with a common graphical user interface of the IMS. Thus users can use the functions of a common graphical user interface to prepare, execute, monitor and view workflows and their data entities. In addition, such a systematic workflow concept supports systematic and complete documentation, easy reusability and automatic execution.

The concept of data entity provides a general possibility to experiment with any data. However, the concept of data entity type makes possible to understand, identify and control the compatibility of different tools. Organization of quantitative data as data sets, each of which comprises a dimensionless variable value matrix, provides maximal compatibility between the data sets and software tools from third parties, because the tools do not have to separate data from dimensions or data descriptors.

Because of the graphical interface, researchers with a biochemical expertise can easily connect the biologically relevant data entities to or from available inputs or outputs and get immediate visual feedback. Inexperienced users can reuse existing workflows to repeat standard workflows merely by changing the input data entities. The requirement to learn the of the syntactic and semantic details of each specific tool's command line can be delegated to technically-qualified persons who integrate new tools to the system. This benefit stems from the separation of the tool definitions from the workflow creation. Biochemical experts can concentrate on workflow creation (defined in terms of data entities, works, workflows, work inputs, workflow inputs work outputs, workflow outputs), while the tool definitions (tools, tool input binders, tool output binders, options, exit codes), are delegated to Information-technology experts.

It is readily apparent to a person skilled in the art that, as the technology advances, the inventive concept can be implemented in various ways. The invention and its embodiments are not limited to the examples described above but may vary within the scope of the claims.

**Acronyms**

IMS: Information Management System
VDL: Variable Description Language
SQL: Structured Query Language
XML: Extendible Markup Language

**Claims**

1. An information management system [="IMS"] for managing workflows, wherein each workflow defines an ordered set of one or more data processing tasks that relate to information (200), wherein the IMS comprises a workflow manager, which comprises:

   - a data entity type definition (1214) for each of several data entity types, wherein each data entity type definition relates to syntax and semantics of data;
   - a data entity definition (1216) for each of several data entities (202, 1250), wherein each data entity relates to a specific data entity type (1214) and contains a subset of the information (200);
   - a tool definition (1208) for each of several tools (1254), wherein each tool is capable of executing a subset of the data processing tasks;
   - one or more tool servers (1222, 1420) for executing the tools;

- a set of tool input binders (1210) and tool output binders (1212), each of which binds an input or output, respectively, of a tool to a specific data entity type;
- a workflow definition (1202, 1202') for each of several workflows, wherein each workflow definition comprises:

    (i) one or more workflow input definitions (1204, 1204'), each workflow input definition indicating one or more data entities as an input (1250) of the workflow;
    (ii) one or more workflow output definitions (1206, 1206'), each workflow output definition indicating one or more data entities as an output (1260) of the workflow;

- wherein the workflow input definitions (1204, 1204') and workflow output definitions (1206, 1206') collectively define a data flow network (1252 - 1258) from the input (1250) of the workflow to the output (1260) of the workflow via one or more instances (1254) of tool definitions (1208).

2. An IMS according to claim 1, wherein the workflow definition (1202) contains a hierarchy (1203), wherein a parent workflow contains several child workflows.

3. An IMS according to claim 1 or 2, further comprising a tool-server binder (1224) for each combination of a tool definition and a tool server.

4. An IMS according to any one of the preceding claims, further comprising a graphical user interface (1300) for creating a visual representation (1352 - 1372) of the data flow network (1252 - 1258) in response to input from a user.

5. An IMS according to claim 4, wherein the graphical user interface comprises a routine for automatically creating a workflow input or output (1204, 1204', 1206, 1206') in response to a user action of connecting an icon of a data entity with an icon of a tool.

6. An IMS according to any one of the preceding claims, further comprising a binder (1230) for defining structured data entities.

7. An IMS according to claim 6, wherein at least some of the structured data entities comprise data sets (610, 620), wherein each data set comprises:

    - a variable value matrix (614, 624) containing variable values organized as rows and columns;
    - a row description list (613, 623), in a variable description language (30), of the rows in the variable value matrix;
    - a column description list (612, 622), in a variable description language (30), of the columns in the variable value matrix;
    - a common factor description (611, 621), in a variable description language (30), of the common factors to all values in the variable value matrix.

8. An IMS according to any one of the preceding claims, further comprising:

    a data entity list definition (1226) for specifying several data entities as an input and/or output of a workflow; and
    a routine for using a tool to process them separately but correlating each input to each output.

9. An IMS according to any one of the preceding claims, further comprising one or more configuration files (1600), each of which comprises:

    an input section (1620, 1630) for defining a tool input binder (1210); and
    an output section (1640) for defining a tool output binder (1212).

10. An IMS according to claim 9, wherein at least one configuration file (1600) also comprises an option section (1650 - 1670) for defining input parameters (1696, 1697) for controlling operation of at least one tool.

11. An IMS according to claim 10, further comprising an installation routine for automatically creating at least some of the data entity type definitions (1214), tool definitions (1208), tool input binders (1210), tool output binders (1212), and option or exit codes (1238, 1239) by using data entity definitions and tool definitions in the one or more configuration files (1600).

12. An IMS according to any one of claims 9 to 11, wherein one or more of the configuration files are extendible markup language files.

# Fig. 1

CT

CT

NW

IMS →

Server                                    S

DB processing
communication
user interface
syntax/logic checks
data processing tools
project management
data tracing
...

DB

# Fig. 2

200

204

Base variables/units

1
*

202

Data set

Contents: variable, biomaterial, time

Organization:
- variable value matrix
- row and column description lists
- fixed dimension description

226

Name
tables

218-1

Gene

218-2

Transcript

218-3

Protein

218-4

Macromolecular
complex

218-5

Compound

218-6

Abiotic stimulus

206

Time    1    *

210-1

Population

210-2

Individual

210-3

Reagent

210-4

Sample

208

Experiment

*
*

*
*

*
*

210

Biomaterial

*

1

220

Database
reference

*

1

218

Biochemical
entity

1

212

Pathway    1    *

*

216

Connection

*

1

214

Location

1

*
1

222

Interaction

1

*

*

224

Kinetic law

214-1    Organism

214-2    Organ

214-3    Tissue

214-4    Cell type

214-5    Cellular
compartment

214-6    Spatial
point

1  *    1-to-many relation
* *    many-to-many relation
→    subclass of

26

Fig. 3A

30

32     33     34     35     36

| Keyword | & | Opening delimiter | & | Name | & | Closing delimiter | & | Separator | & | 31 |

31

Fig. 3B

| 38 | 38' | 38" |
|---|---|---|
| T | Time (relative) | T[-2.57E-3] |
| Ts | Time stamp | Ts[2002-11-26 14:00:00] |
| Sa | Sample | Sa[123456] |
| Po | Population | Po[2233445] |
| Id | Individual | Id[patient xyz] |
| Re | Reagent | Re[reagent a] |
| L | Location | L[yeast K2 mitocondria] |
| X/Y/Z | Spatial Z/Y/Z coordinate | X[0] relative to reference model |
| O | Organism | O[human] |
| V | Variable | V[concentration] |
| U | Unit | U[mol/l] |
| G | Gene | G[P53] |
| Tr | Transcript | Tr[mRNA from gene P53 to protein P53] |
| P | Protein | P[P53] |
| C | Compound | C[mannose] |
| M | Macromolecular complex | M[ribosome] |
| A | Abiotic stimuli | A[light] |
| I | Interaction | I[EC 1.2.3.4_P12345_F] |

Fig. 3C

401 —— V[concentration] of C[mannose]in U[mol/l]

402 —— V[concentration] C[mannose]U[mol/l]

403 —— V[concentration]of O[rat]P[P53] in U[mol/l]

404 —— V[concentration] O[rat]P[P53] U[mol/l]

405 —— V[concentration]of O[human]P[P53] in L[rat nucleus] in U[mol/l]

406 —— V[concentration]O[human]P[P53] L[rat nucleus] U[mol/l]

407 —— V[flux] of C[mannose]from I[EC 2.7.7.14 PSA1] in U[mol/l]

408 —— V[flux]C[mannose]I[EC 2.7.7.14-PSA1]U[mol/l/s]

409 —— V[reaction rate] through I[EC 2.7.7.14-PSA1] in U[mol/l/s]

410 —— V[reaction rate]I[EC 2.7.7.14-PSA1]U[mol/l/s]

411 —— I[EC 2.7.7.14-PSA1]

412 —— V[flux] L[yeast golgi] C[mannose]I[mannose transport]U[mol/l/s]

414 —— V[Expression level]Tr[mRNA from gene P53 to protein P53]U[-]

414 —— V[*]P[*]0[*]L[*]U[*]

415 —— Ts[2003.1.2 10:00:00, 2003.1.2 14:00:00]

417 —— Ts[2003.1.2 12:00:00]T[-5,0, 5.0]

418 —— L[human_eyelid_epith_nuc]

Fig. 4

Fig. 5

500 →

Variable

Biomaterial

Yeast population abcd1234

Time 2003.06.10 12:30:00          Time

511

510 ⌒ V[concentration]C[mannose]U[mol/l]Po[abcd1234]Ts[2003.06.10 12:30:00]=0.014

Variable

Biomaterial

521

Time

520 ⌒ V[concentration]C[mannose]U[mol/l]Po[abcd1234]

Variable

Biomaterial

531

Time

530 ⌒ V[concentration]C[mannose]U[mol/l]Ts[2003.06.10 12:30:00]

Variable

Biomaterial

542

541

Time

540 ⌒ V[concentration]C[mannose]U[mol/l]

**Fig. 6A**

610

611

| Ts[*] |
|---|

| V[expression level]Tr[mRNA1...]U[-] |
|---|
| V[expression level]Tr[mRNA2...]U[-] |
| V[expression level]Tr[mRNA3...]U[-] |
| V[expression level]Tr[mRNA4...]U[-] |
| V[expression level]Tr[mRNA5...]U[-] |
| V[expression level]Tr[mRNA6...]U[-] |
| ... |
| V[age]U[years] |

613

612

| Id[patient A] | Id[patient B] | Id[patient C] | Id[patient D] |
|---|---|---|---|
| 25.18 | 40.83 | 39.46 | 28.15 |
| 9.76 | 15.45 | 14.91 | 11.84 |
| 250.66 | 99.43 | 546.23 | 429.00 |
| 99.02 | 75.39 | 88.25 | 94.67 |
| 3.07 | 9.45 | 10.23 | 6.78 |
| 101.12 | 210.85 | 124.92 | 145.63 |
| ... | | | |
| 67 | 81 | 75 | 62 |

614

615

```
Expression level of mRNA1 - mRNA6 of patients A - D at (time)
                A            B            C            D
mRNA1   25.18        40.83        39.46        28.15
mRNA2    9.76        15.45        14.91        11.84
mRNA3  250.66        99.43       546.23       429.00
mRNA4   99.02        75.39        88.25        94.67
mRNA5    3.07         9.45        10.23         6.78
mRNA6  101.12       210.85       124.92       145.63
                ...
age        67           81           75           62
```

**Fig. 6B**

620

621

| Po[Saccharomyces cerevisiae]<br>Sa[xyz]Ts[2003.01.15 12:10:00] |
|---|

| V[expression level]Tr[mRNA1...]U[-] |
|---|
| V[expression level]Tr[mRNA2...]U[-] |
| V[expression level]Tr[mRNA3...]U[-] |
| V[expression level]Tr[mRNA4...]U[-] |
| V[expression level]Tr[mRNA5...]U[-] |
| V[expression level]Tr[mRNA6...]U[-] |
| ... |
| V[temperature]U[centigrade] |

623

622

| T[0] | T[30] | T[60] | T[120] |
|---|---|---|---|
| 2.8 | 4.3 | 9.6 | 12.5 |
| 90.6 | 53.5 | 14.91 | 15.8 |
| 2500.6 | 1000.3 | 546.3 | 100.0 |
| 99.2 | 75.5 | 68.5 | 194.2 |
| 300.0 | 259.4 | 102.3 | 117.7 |
| 191.2 | 214.8 | 412.9 | 450.3 |
| ... | | | |
| 25.0 | 30.1 | 35.0 | 39.8 |

624

**Fig. 6C**

630

| Keyword | Value | Row | Column | (ObjectClass)/(ObjectID) |
|---|---|---|---|---|
| Po | Saccharomyces cerevisiae | -1 | -1 | |
| Sa | xyz | -1 | -1 | |
| Ts | 2003.01.15 12:10:00 | -1 | -1 | |
| V | expression level | 1-6 | -1 | |
| Tr | mRNA1 | 1 | -1 | |
| Tr | mRNA2 | 2 | -1 | |
| Tr | mRNA3 | 3 | -1 | |
| Tr | mRNA4 | 4 | -1 | |
| Tr | mRNA5 | 5 | -1 | |
| Tr | mRNA6 | 6 | -1 | |
| U | - | 1-6 | -1 | |
| V | temperature | 8 | -1 | |
| U | centigrade | 8 | -1 | |
| T | 0 | -1 | 1 | |
| T | 30 | -1 | 2 | |
| T | 60 | -1 | 3 | |
| T | 120 | -1 | 4 | |

631 (Po, Sa, Ts)
633 (V through U/centigrade)
632 (T rows)

634 — 634A 634B 634C

| | | |
|---|---|---|
| 1 | 1 | 2.8 |
| 1 | 2 | 4.3 |
| 1 | 3 | 9.6 |
| 1 | 4 | 12.5 |
| 2 | 1 | 90.6 |
| ... | | |
| 8 | 3 | 35.0 |
| 8 | 4 | 39.8 |

Fig. 7A

700

217
Connection

212
Pathway

214
Location

222
Interaction

218
Biochemical entity

224
Kinetic law

Common biochemical entity

Main_pathway
From_pathway
To_pathway

702
Pathway connection

Common interaction

Fig. 7B

711
Pathway   A

Entity | Connection | Interaction

Main

721

720
Pathway connection

From
722

To
723

725
734

730
Pathway connection
From   Main

To

712   724

Pathway   B

Entity | Connection | Interaction

735   714

Pathway   C

Entity | Connection | Interaction

Fig. 7C

744
G[A]

745
P[B]

743

C[RNA]

X

Tr[mRNA]

741

742

Y

746

Fig. 8

*800*

*810* PSA1

V[reaction rate]U[mol/l/s]I[EC 2.7.7.14_PSA1]=0.01

*882*

*883*

V[flux]U[mol/l/s]...
C[GDP-D-mannose]...
I[EC 2.7.7.14_PSA1]=0.01

GTP *820*

Pyrophosphate

D-mannose 1-phosphate

EC 2.7.7.14_PSA1

*860*

GDP-D-mannose

*881*

V[concentration]U[mol/l]...
C[GDP-D-mannose]=1.0

GDP

EC 2.7.7.22_GMPPB

Orthophosphate

*850* GMPPB *840*

| | | |
|---|---|---|
| *810* → ◯ | Biochemical entity | |
| *820* → ▭ | Interaction | |
| *830* → ⌐ ⌐ ← | Connections | |
| *840* → ◯ → ⌐ ⌐ | Substrate connection, interaction consumes biochemical entity | |
| *850* → ⌐ ⌐ → ◯ | Product connection, interaction produces biochemical entity | |
| *860* → ----▸ | Activation | |
| *870* → ----⊣ | Inhibition | |

Fig. 9A

*900*

*902* Project

Input deliverables

*906* 1 *904* 1 *908* Output deliverables

Equipment 1 * Experiment (general activity) * 1 User

*916* *914* *910* *920* *922*

Biomaterial 1 * Experiment input * 1 Method 1 Experiment output * 1 Biomaterial

*918* *924*

Data entity 1 1 1 Data entity

*915* *930* *912* *921*

Time 1 Experiment target Method description 1 Time

*932* *934*

Biomaterial Data entity

# Fig. 9B

Perturbation enters a location. Sample taken from a location.
Location = L[fe-yeast_ext] = En[fermentor], O[yeast], Cc[extra_cellular]

Fig. 10

1002 Condition
1004 Phenotype
1006 Data entity

210 Biomaterial

210-1 Population
210-2 Individual
210-3 Reagent
210-4 Sample

1008 Organism binder

214 Location

Organism 214-1
Organ 214-2
Tissue 214-3
Cell type 214-4
Cellular compartment 214-5

1010

⇧ Subclass of

1010B

Bacteria
└ Escherichia coli
    └ Escherichia coli K-12 MG1755
        └ Escherichia coli K-12 MG1755 x

1010B

Eukaryota
└ Viridiplantae
    └ Streptophyta
        └ Embryophyta
            └ Tracheophyta
                └ Spermatophyta
                    └ Magnoliophyta
                        └ eudicotyledons
                            └ core eudicots
                                └ Rosidae
                                    └ eurosids I
                                        └ Fabales
                                            └ Fabaceae
                                                └ Papilionoideae
                                                    └ Trifolieae
                                                        └ Trifolium
                                                            └ Trifolium repens
                                                               (white clover)

## Fig. 11A

Id[A]  1102

1104

T[0] → Sa[1]T[0]=Id[A]T[0]

T[0] → Sa[3]T[0]=Id[A]T[0]

T[5] → Sa[4]T[5]=Id[A]T[5]   1106   1108

T[12] → Sa[25]L[nucleus]T[12]=Sa[4]L[nucleus]T[5]=Id[A]L[nucleus]T[5]   1110

T[5] → Sa[6]T[5]=Id[A]T[5]

1112

V[concentration]P[P53]Sa[25]L[nucleus]T[12]=
V[concentration]P[P53]Id[A]L[nucleus]T[5]=4.95

T[12]

Sa[27]T[12]=Sa[4]T[5]=Id[A]T[5]

V[concentration]P[P53]Sa[27]T[12]=
V[concentration]P[P53]Id[A]T[5]=3.24

## Fig. 11B

1152   1170

V[dose]U[g]C[abcd]Sa[40]T[1]=
V[dose]U[g]C[abcd]Id[B]T[6]=10

V[dose]U[g]C[mannose]Sa[40]T[0]=
V[dose]U[g]C[mannose]Id[B]T[5]=10.0

1154 — T[1]

Sa[40]T[1]   1156

T[0]

Sa[40]T[0]

1158 — T[6]   1150

Id[B]

T[5]

1180

T[7] → Sa[7]T[7]

T[7] → Sa[9]T[7]

T[10] → Sa[10]T[10]   1182

T[10] → Sa[12]T[10]=Id[B]T[10]

1184

V[concentration]P[P53]Sa[12]T[10]=
V[concentration]P[P53]Id[B]T[10]=9.34

## Fig. 12A

1218 — Project
1220 — User
1222 — Server DB

1202
1203
Workflow

1224 — Tool-server binder

1208 — Tool

1204 — Workflow input
1210 — Tool input binder
1214 — Data entity type
1212 — Tool output binder
1206 — Workflow output

1216 — Data entity

| File | Folder/file | DB/Object/attribute | URL/URI | ⋯ | Other form of data |

1200

## Fig. 12B

Graphical workflow editor
1240
CT

Workflow database — 1246

Workflow server:
- Workflow manager
- Database API
1242

Tool server:
- Executor
- Service object
- Tools
1244

## Fig. 12C

1260

1250 — Data entity 1
1252
1254 — Tool X
1256 — Data entity 3
1258 — Data entity 4
Data entity 2

Tool Y — 1256
Data entity 5
Tool Z — 1254
Data entity 6
Data entity 7
1252

## Fig. 12D

# Fig. 13

1300

1302 — Workflow editor - [O:\Workflows\Demos\Workflow_demo]

1304 — Workflow  Edit  Tool  In/out  Data set...

1310 —
ABC Database Populator
Digest
Hierarchical Clustering
K-means Clustering
MS Composition Fitter
Normalization
...

1320 —
Tool description
Fits composition to MS data

1330 —
| Tool inputs | Data type |
|---|---|
| input 1 | (type) |
| input 2 | (type) |
| ... | ... |

1340 —
| Tool outputs | Data type |
|---|---|
| output 1 | (type) |
| output 2 | (type) |
| ... | ... |

1350

1372 — data in MS composition fit test XYZ 2003-12-05
1)output
1362 — MS Composition Fitter
1364 — 4)digest
1358 — Plmn_digest
1360 — 1)output report
1354 — Digest
1356 — 1)input sequence
1352 — Plmn_human

1366 — Compositions
1)compositions
1368 — MS data
2)MS data
1370 — Parameters 2
3)Parameters

Execution status  OK
Exit message  Executed successfully

39

Fig. 14

Workflow
editor

is interface for

1406

1400

Application server

Workflow
executer

1402

Resource
handler

1404

1450

Remote
executer

is interface for

1420

Tool server

Tool server

1422

uses for
execution
of tools

is interface for

uses for getting
data entities

<<ejb_object>>
Resource handler

Service object

<<rmi_object>>
Organization resource manager

is interface for

File server

File server

1440

1442

Fig. 15

| Tool server | Context | Remote executer | Workflow handler |
|---|---|---|---|

15-1  Read configuration

15-2  Create context for tool server

15-3  Create object

15-4  Rebind tool server to context

15-5  Register tool server

**Fig. 16A**

1600

```
<?xml version="1.0" encoding="iso-8859-1" ?>
<! --------------------------
// (c) 2003 Medicel Oy, revision 1.1
// $Header: /var/cvsroot/bms/com/medicel/bms/tool/emboss/digest.xml, v 1.2
// Description: xml for digest
-------------------------------->
<tool-definition>
    <tool
        tool_name="Digest"
        description="Digest finds positions where a specified
            proteolytic enzyme or reagent may cut a peptide sequence."
        executable ="digest"
        number_of_inputs="2"
        number_of_outputs="1"
        source="Emboss"
        category="Protein:motifs" >
        <!-- input section -->
        <input
            pre_tag="-sequence "
            name="Input sequences"
            description="Input sequences entity."
            number="1"
            command_line_order="1"
            data_entity_type="DT-SEQ-AA-FASTA"
            optional="0" />
        <input
            pre_tag=""
            name="Options"
            description="Options."
            number="2"
            data_entity_type="DT-OPTIONS"
            option_replacement="1"
            optional= "1" />

        <!-- output section -->
        <output
            pre_tag="-outfile "
            name="Output report"
            description="Output report entity."
            number="1"
            command_line_order="2"
            data_entity_type="DT-EMBOSS-REPORT"
            optional="0" />
```

1605

1610A

1612A

1615

1620

1630

1640

**Fig. 16C**

1690

1691 1692 1693 1694 1695 1696 1697

Digest -sequence myinfile -outfile myoutfile -auto -unfavoured

**Fig. 16B**

1600

```
        <!-- options section -->
        <option
                pre_tag="-auto"
                name=""
                description=""
                command_line_order="3"
                data_type=""
                data_default_value=""
                optional="0" />
        <option
                pre_tag="-menu"
                name="menu"
                description=""
                command_line_order="4"
                values="1 Trypsin:2 LyS-C:3 Arg-C:4 ASP-N:5 V8-bicarb:6 V8-phosph:7+
                  Chymotrypsin:8 CNBr"
                optional="1" />
        <option
                pre_tag="-unfavoured"
                name="unfavoured"
                description="Trypsin will not normally cut after a K if followed by eg another K or a P.+
                  Specifying this shows those cuts as well as the favoured ones. Do not give value."
                command_line_order="5"
                optional="1" />
        <option
                pre_tag="-overlap"
                name="overlap"
                description="Used for partial digestion. Shows all cuts from favoured cut sites plus+
                  1.3, 2-4, 3-5 etc but not eg 2-5. Overlaps are therefore fragments with exactly+
                  one potential cut site within it. Do not give value."
                command_line_order="6"
                optional="1" />
        <option
                pre_tag="-allpartials"
                name="alllpartials"
                description="As for overlap but fragments containing more than one potential cut+
                  site are included. DO NOT give any value."
                command_line_order="7"
                optional="1" />
        <!-- error codes -->
        <error-codes
                error-condition="ne"
                limit="0" >
                <exit-code
                        number="0"
                        short_desc="Done."
                        description="Successfully done." />
        </error-codes>
        </tool>
</tool-definition>
```

1650
1655
1660
1665
1670
1680
1612B
1610B

Fig. 17A

EP 1 494 156 A2

**WorkflowExecuter**

**RemoteExecutionListener**

+executionFinished(handle:int, warnings: boolean)
+executionFinishedToError(handle:int)
+processProgressed(handle:int, progress:float)

**WorkflowExecuterSB**

+execute(workflow:int)
+execute(so)
+stopExecution(workflow:int)
+executionFinished(handle:int, warnings: boolean)
+executionFinishedToError(handle:int)
+processProgressed(handle:int, progress:float)
+publish(parentId:int):int
-scanGraph(parentId:int):int
-createSO(wf:graph)
-createGraph(workflowid:int):WorkflowGraph

**ExecutionStatus**

+putInExecution(so,re)
+getRemoteExecuter()
+executionFinished(handle:int)
+getServiceObjectByProperty(key:string, regex:string)

Starts

1    1

**<<thread>>**
**ExecutionThread**

+run()

1    1

**WorkflowExecuter**

1

0 - *

1

**TopicPublisher**

+publish(message:message)

1    1

**ServiceObjectQueue**

+add(so)
+get()
+isEmpty():boolean
+removeByProp(key:string, regex:string)

(Fig. 17B)

# Fig. 17B

ToolServer

**Remote**

1720

**RemoteExecuter**

+executeAsync(so:ServiceObject)
+stopExecution(handle:int)
+getHostName():string
+getNumberOfToolsRunning():int
+canExecuteMore():int

1710

**ServiceObject**

+execute()
+stopExecution()
+getPriority():int
+setClientProperty(key:string):string
+getHnadle():int
+setHnadle(h:int)
+addListener(l)

1711

uses for
getting
workflows

**Workflow**

○

<ejb object>
WorkflowManager

uses for
getting
data entities

**ResourceHandler**

○

<ejb object>
ResourceHandler

1730

**ToolServer**

+main()
+readConfiguration()

1

1740

1741

**WorkflowServiceObject**

+initialize(wf)
+execute()

**CommandLineServiceObject**

EP 1 494 156 A2

44

Fig. 18

EP 1 494 156 A2

| Client | WorkflowExecuterSB | ServiceObjectQueue | <<thread>> ExecutionThread | ExecutionStatus | RemoteExecuter (re) | ServiceObject (so) |

Execute(workflow)
18-2

CreateGraph(workflow)
18-4

ShowGraph(parentId)
18-6

For each 'free' service object

18-8

Add(so)
18-10

Get()
GetRemoteExecuter() 18-14
18-12

PutInExecution(so,re) 18-16

ExecuteAsync(so)
18-18

Execute 18-20

18-22

ExecutionFinished(handle,warnings)

Start from step 18-6. Stop if no more elements.